# EUROPEAN PATENT APPLICATION

(11) **EP 2 410 060 A1**
(43) Date of publication of application: **25.01.2012**
(21) Application number: 11185683.7
(22) Date of filing: 22.08.2001
(51) Int. Cl.: C12N 15/82, C07K 14/415, A01H 5/00

(54) **Genes for modifying plant traits IV**

(30) Priority: 22.08.2000 US 227439 P; 16.11.2000 US 713994; 18.04.2001 US 837944
(62) Divisional of application: 01966075.2
(71) Applicant: Mendel Biotechnology, Inc., Hayward, CA 94545 (US)
(72) Inventor: Pilgrim, Marsha L., Phoenixville, PA 19460 (US); Riechmann, Jose Luis, 08950 Barcelona (ES); Yu, Guo-Liang, Berkeley, CA 94705 (US); Pineda, Omaira, Vero Beach, FL 32960 (US); Creelman, Robert A., Castro Valley, CA 94546 (US); Dubell, Arnold N., San Lorenzo, CA 94580 (US); Heard, Jacqueline E., Wenham, MA 01984 (US); Jiang, Cai-Zhong, Davis, CA 95618 (US); Keddie, James S., San Mateo, CA 94402 (US); Adam, Luc J., Hayward, CA 94545 (US); Ratcliffe, Oliver, Oakland, CA 94606 (US); Reuber, T. Lynne, San Mateo, CA 94402 (US)
(74) Representative: Brasnett, Adrian Hugh

(57) **Abstract**

The invention relates to plant transcription factor polypeptides, polynucleotides that encode them, homologs from a variety of plant species, and methods of using the polynucleotides and polypeptides to produce transgenic plants having advantageous properties compared to a reference plant. Sequence information related to these polynucleotides and polypeptides can also be used in bioinformatic search methods and is also disclosed.

## Description

This application claims priority benefit of: prior U.S application entitled "Plant Trait Modification III," serial no. 60/227,439, filed August 22, 2000; prior U.S. application entitled "Genes for Modifying Plant Traits," attorney docket number MBI-0022, serial no. ______, filed November 16, 2000; and prior U.S. application entitled "Genes for Modifying Plant Traits II," serial no. 09/837,944, filed April 18, 2001. The entire content of each of these applications is hereby incorporated by reference.

### Field of the Invention and Introduction

This invention relates to the field of plant biology. More particularly, the present invention pertains to compositions and methods for phenotypically modifying a plant.

A plant's traits, such as its biochemical, developmental, or phenotypic characteristics, can be controlled through a number of cellular processes. One important way to manipulate that control is through transcription factors - proteins that influence the expression of a particular gene or sets of genes. Transgenic plants that comprise cells having altered levels of at least one selected transcription factor, for example, possess advantageous or desirable traits. Strategies for manipulating traits by altering a plant cell's transcription factor content can therefore result in plants and crops with commercially valuable properties. Applicants have identified polynulceotides encoding transcription factors, developed numerous transgenic plants using these polynucleotides, and have analyzed the plants for a variety of important traits. In so doing, applicants have identified important polynucleotide and polypeptide sequences for producing commercially valuable plants and crops as well as the methods for making them and using them. Other aspects and embodiments of the invention are described below and can be derived from the teachings of this disclosure as a whole.

### Background of the Invention

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

The present invention provides novel transcription factors useful for modifying a plant's phenotype in desirable ways.

### Summary of the Invention

In a first aspect, the invention relates to a recombinant polynucleotide comprising a nucleotide sequence selected from: (a) a nucleotide sequence of the Sequence Listing, or SEQ ID Nos.: 2N-1 where N=1-232, preferably where N=1-232, or a nucleotide sequence encoding a polypeptide comprising an amino acid sequence selected from those of the Sequence Listing, or SEQ ID Nos: 2N where N=1-232, or a complementary nucleotide sequence of any of these; (b) a nucleotide sequence encoding a polypeptide comprising a variant of a polypeptide of (a) or a variant having one or more, or between 1 and about 5, or between 1 and about 10, or between 1 and about 30, conservative amino acid substitutions; (c) a nucleotide sequence comprising a sequence selected from SEQ ID Nos.: 2N-1 where N=1-232, or a complementary nucleotide sequence thereof; (d) a nucleotide sequence comprising one or more silent substitutions in a nucleotide sequence of (c); (e) a nucleotide sequence that hybridizes under stringent conditions, high stringent conditions, ultra-high stringent conditions, or ultra-ultra-high stringent conditions over substantially the entire length of a nucleotide sequence of one or more of (a), (b), (c), or (d); (f) a nucleotide sequence comprising at least 10 or 15, or at least about 20, or at least about 30 consecutive nucleotides of a sequence of any of (a)-(e), or at least 10 or 15, or at least about 20, or at least about 30 consecutive nucleotides outside of a region encoding a conserved domain of any of (a)-(e); (g) a nucleotide sequence comprising a subsequence or fragment of any of (a)-(f), which subsequence or fragment encodes a polypeptide having a biological activity that modifies a plant's characteristic, functions as a transcription factor, results in ectopic expression or altered expression in a transgenic plant, or alters the level of transcription of a gene or transgene in a cell; (h) a nucleotide sequence having at least 31% sequence identity to a nucleotide sequence of any of (a)-(g); (i) a nucleotide sequence having at least 60%, or at least 70 %, or at least 80 %, or at least 90 %, or at least 95 % sequence identity to a nucleotide sequence of any of (a)-(g) or a 10 or 15 nucleotide, or at least about 20, or at least about 30 nucleotide region of a sequence of (a)-(g) that is outside of a region encoding a conserved domain; (j) a nucleotide sequence that encodes a polypeptide having at least 31% sequence identity to a polypeptide listed in the Sequence Listing, or SEQ ID No.: 2N-1 where N=1-232; (k) a nucleotide sequence that encodes a polypeptide having at least 60%, or at least 70 %, or at least 80%, or at least 90 %, or at least 95 % sequence identity to a polypeptide listed in the Sequence Listing, or SEQ ID No.: 2N-1 where N=1-232; and (1) a nucleotide sequence that encodes a conserved domain of a polypeptide having at least 85%, or at least 90%, or at least 95%, or at least 98% sequence identity to a conserved domain of a polypeptide listed in the Sequence Listing, or SEQ ID No.: 2N-1 where N=1-232. A recombinant polynucleotide may further comprise a constitutive, inducible, or tissue-specific promoter operably linked to a nucleotide sequence listed above. The invention also relates to compositions comprising at least two of the above-described polynucleotides.

In a second aspect, the invention comprises an isolated or recombinant polypeptide having an amino acid sequence of the Sequence Listing, or SEQ ID Nos.: 2N-1 where N=1-232, or a polypeptide comprising a subsequence of at least about 10, or at least about 15, or at least about 20, or at least about 30 contiguous amino acids encoded by the recombinant or isolated polynucleotide described above, or comprising a subsequence of at least about 8, or at least about 12, or at least about 15, or at least about 20, or at least about 30 contiguous amino acids outside of a conserved domain.

In another aspect, the invention is a transgenic plant comprising one or more of the above-described recombinant polynucleotides. In yet another aspect, the invention is a plant with altered expression levels of a polynucleotide described above or a plant with altered expression or activity levels of an above-described polypeptide. Further, the invention is a plant lacking a nucleotide sequence encoding a polypeptide described above or substantially lacking a polypeptide described above. The plant may be any appropriate plant, including, but not limited to, *Arabidopsis*, mustard, soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, sugarbeet, canola, peanut, rosaceous fruits, vegetable brassicas, and mint or other labiates.

In a further aspect, the invention relates to a cloning or expression vector comprising the isolated or recombinant polynucleotide described above or cells comprising the cloning or expression vector.

In yet a further aspect, the invention relates to a composition produced by incubating a polynucleotide of the invention with a nuclease, a restriction enzyme, a polymerase, a polymerase and a primer, a cloning vector, or with a cell.

Furthermore, the invention relates to a method for producing a plant having a modified trait. The method comprises altering the expression of an isolated or recombinant polynucleotide of the invention or altering the expression or activity of a polypeptide of the invention in a plant to produce a modified plant, and selecting the modified plant for a modified trait.

In another aspect, the invention relates to a method of identifying a factor that is modulated by or interacts with a polypeptide encoded by a polynucleotide of the invention. The method comprises expressing a polypeptide encoded by the polynucleotide in a plant and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

In yet another aspect, the invention is a method of identifying a molecule that modulates activity or expression of a polynucleotide or polypeptide of interest. The method comprises placing the molecule in contact with a plant comprising the polynucleotide or polypeptide encoded by the polynucleotide of the invention and monitoring one or more of the expression level of the polynucleotide in a cell of the plant, the expression level of the polypeptide in a cell of the plant, and the modulation of an activity of the polypeptide in a cell of the plant.

In yet another aspect, the invention relates to an integrated system, computer or computer readable medium comprising one or more character strings corresponding to a sequence of the Sequence Listing, SEQ ID Nos.: 1-464, to a polynucleotide of the invention, or to a polypeptide encoded by the polynucleotide. The integrated system, computer or computer readable medium may comprise a link between one or more sequence strings to a modified plant trait.

In yet another aspect, the invention is a method for identifying a sequence similar to or homologous to one or more polynucleotides of the invention, or one or more polypeptides encoded by the polynucleotides. The method comprises providing a sequence database, and querying the sequence database with one or more target sequences corresponding to the one or more polynucleotides or to the one or more polypeptides, such as those of SEQ ID Nos.: 1-464, to identify one or more sequence members of the database that display sequence similarity or homology to one or more of the one or more target sequences. Such a method may also be a method of identifying a homolog sequence from a database, where the database comprises a plurality of known plant sequences. These sequences can be ESTs, cDNA, or genomic fragments. The database may contain sequences that are not "known" in addition to the known sequences, in that sequences may not be assigned or linked to a function or particular characteristic, yet the sequence itself is known. The method of identifying a homolog comprises inputting sequence information selected from one or more of SEQ ID Nos. 1-464; and querying the database to identify a homolog sequence. In this way, homolog sequences from any number of plant species, cultivars, or strains can be identified from the information of an inputted sequence or a fragment of the sequence. For these methods and for the sequence information, a computer readable medium having stored sequence information of one or more of SEQ ID Nos.: 1-464, or 1-37, or any one particular SEQ ID No., or any group of SEQ ID Nos. in between 1 and 464, can be used. The computer readable medium may include, for example, a floppy disc, a hard drive, random access memory (RAM), read only memory (ROM), and/or CD-ROM.

A method of the invention may comprise linking the one or more of the polynucleotides of the invention, or encoded polypeptides, to a modified plant phenotype.

### Brief Description of the Sequence Listing and the Appendices

The Sequence Listing provides exemplary polynucleotide (SEQ ID Nos.: 2N-1 where N=1-232) and polypeptide (SEQ ID Nos.: 2N where N=1-232) sequences of the invention. The traits associated with the use of the sequences are included in the Examples or the Appendices.

The Tables of the Appendices include homologous sequences and homologs of specific polynucleotides and polypeptides, specific information about those sequences, and data concerning exemplary transgenic plants of the invention. The data and sequence information can be prepared according to the methods of the Examples or those known in the art. The Appendices include the Tables of this Appendix and those in the files of the Appendices of the priority documents.

Table 3 in the Appendix is a list of: the first 332 sequences from the Sequence Listing; the corresponding GID number (i.e. G28) used throughout to refer to both the cDNA and protein sequence of a particular transcription factor, and referred to or used in the Appendices of the U.S. priority documents; and the identification of conserved amino acid domain start and stop sites (conserved domain) within the protein sequence.

Table 4 in the Appendix is a list of: selected sequences from the Sequence Listing; their corresponding GID number; the type of transgenic plant produced to determine ectopic expression, altered expression, or trait (either Knockout of overexpressor as in the Examples); and general descriptions and specific characteristics of the transgenic plant's traits as compared to a wild type, reference, or control plant.

Table 5 of the Appendix is a list of: selected sequences from the Sequence Listing; their corresponding GID number; the identification of the one or more homolog sequences and the corresponding GID numbers; the type of sequence of the particular SEQ ID No.; and the identification of conserved amino acid domain start and stop sites (conserved domain) within the protein sequence.

Table 6 of the Appendix is a list of selected homologs identified from genomic, EST, or other database, as referred to in the Examples. Table 6 includes: the particular SEQ ID No. in the Sequence Listing used to identify exemplary homologs; the corresponding GID number of the SEQ ID No. sequence; the Genbank NID reference number associated with the exemplary homolog identified (from which one of skill in the art can produce a genomic, cDNA, and/or EST sequence and corresponding polynucleotide); the P-value related to the particular, exemplary homolog comparison to the GID sequence; the percent identity between the GID sequence and the homolog; and the species the exemplary homolog sequence is derived from. All of the sequences referred to in the Table, as well as fragments or parts of these sequences, can be used in accordance with this invention, for example to produce transgenic plants with ectopic expression or altered expression.

### Detailed Description of Exemplary Embodiments

In an important aspect, the present invention relates to polynucleotides and polypeptides, e.g. for modifying phenotypes of plants. Throughout this disclosure, various information sources are referred to and/or are specifically incorporated. The information sources include scientific journal articles, patent documents, textbooks, and web pages, for example. While the reference to these information sources clearly indicates that they can be used by one of skill in the art, applicants specifically incorporate each and every one of the information sources cited herein, in their entirety, whether or not a specific mention of "incorporation by reference" is noted. The contents and teachings of each and every one of the information sources can be relied on and used to make and use embodiments of the invention.

The polynucleotides of the invention encode plant transcription factors or fragments of them. As one of ordinary skill in the art recognizes, transcription factors can be identified by the presence of a region or domain of structural similarity or identity to a specific consensus sequence or the presence of a specific consensus DNA-binding site (*see*, *for example*, Riechmann et al., Science 290: 2105-2110 (2000)). The plant transcription factors may belong to one of the following transcription factor families: the AP2 (APETALA2) domain transcription factor family (Riechmann and Meyerowitz (1998) Biol. Chem. 379:633-646); the MYB transcription factor family (Martin and Paz-Ares, (1997) Trends Genet. 13:67-73); the MADS domain transcription factor family (Riechmann and Meyerowitz (1997) Biol. Chem. 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the zinc finger protein (Z) family (Klug and Schwabe (1995) FASEB J. 9: 597-604); the homeobox (HB) protein family (Duboule (1994) Guidebook to the Homeobox Genes, Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 250:7-16); the NAM protein family (Souer et al. (1996) Cell 85:159-170); the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the Box P-binding protein (the BPF-1) family (da Costa e Silva et al. (1993) Plant J. 4:125-135); the high mobility group (HMG) family (Bustin and Reeves (1996) Prog. Nucl. Acids Res. Mol. Biol. 54:35-100); the scarecrow (SCR) family (Di Laurenzio et al. (1996) Cell 86:423-433); the GF14 family (Wu et al. (1997) Plant Physiol. 114:1421-1431); the polycomb (PCOMB) family (Kennison (1995) Annu. Rev. Genet. 29:289-303); the teosinte branched (TEO) family (Luo et al. (1996) Nature 383:794-799; the ABI3 family (Giraudat et al. (1992) Plant Cell 4:1251-1261); the triple helix (TH) family (Dehesh et al. (1990) Science 250:1397-1399); the EIL family (Chao et al. (1997) Cell 89:1133-44); the AT-HOOK family (Reeves and Nissen (1990) Journal of Biological Chemistry 265:8573-8582); the S1FA family (Zhou et al. (1995) Nucleic Acids Res. 23:1165-1169); the bZIPT2 family (Lu and Ferl (1995) Plant Physiol. 109:723); the YABBY family (Bowman et al. (1999) Development 126:2387-96); the PAZ family (Bohmert et al. (1998) EMBO J. 17:170-80); a family of miscellaneous (MISC) transcription factors including the DPBF family (Kim et al. (1997) Plant J. 11:1237-1251) and the SPF1 family (Ishiguro and Nakamura (1994) Mol. Gen. Genet. 244:563-571); the golden (GLD) family (Hall et al. (1998) Plant Cell 10:925-936), the TUBBY family (Boggin et al, (1999) Science 286:2119-2125), the heat shock family (Wu C (1995) Annu Rev Cell Dev Biol 11:441-469), the ENBP family (Christiansen et al (1996) Plant Mol Biol 32:809-821), the RING-zinc family (Jensen et al. (1998) FEBS letters 436:283-287), the PDBP family (Janik et al Virology. (1989) 168:320-329), the PCF family (Cubas P, et al. Plant J. (1999) 18:215-22), the SRS (SHI-related) family (Fridborg et al Plant Cell (1999) 11:1019-1032), the CPP (cysteine-rich polycomb-like) family (Cvitanich et al Proc. Natl. Acad. Sci. U S A. (2000) 97:8163-8168), the ARF (auxin response factor) family (Ulmasov, et al. (1999) Proc. Natl. Acad. Sci. USA 96: 5844-5849), the SWI/SNF family (Collingwood et al J. Mol. End. 23:255-275), the ACBF family (Seguin et al Plant Mol Biol. (1997) 35:281-291), PCGL (CG-1 like) family (Plant Mol Biol. (1994) 25:921-924) the ARID family (Vazquez et al Development. (1999) 126: 733-42), the Jumonji family, Balciunas et al (Trends Biochem Sci. (2000) 25: 274-276), the bZIP-NIN family (Schauser et al Nature. (1999) 402: 191-195), the E2F family Kaelin et al (1992) Cell 70: 351-364) and the GRF-like family (Knaap et al (2000) Plant Physiol. 122: 695-704. As indicated by any part of the list above and as known in the art, transcription factors have been sometimes categorized by class, family, and sub-family according to their structural content and consensus DNA-binding site, for example. All of the classes and many of the families and sub-families are listed here. However, the inclusion of one sub-family and not another, or the inclusion of one family and not another, does not mean that the invention does not encompass polynucleotides or polypeptides of a certain family or sub-family. The list provided here is merely an example of the types of transcription factors and the knowledge available concerning the consensus sequences and DNA-binding site motifs that help define them (each of the references noted above are specifically incorporated herein by reference).

In addition to methods for modifying a plant phenotype by employing one or more polynucleotides and polypeptides of the invention described herein, the polynucleotides and polypeptides of the invention have a variety of additional uses. These uses include their use in the recombinant production (i.e, expression) of proteins; as regulators of plant gene expression; as diagnostic probes for the presence of complementary or partially complementary nucleic acids (including for detection of natural coding nucleic acids); as substrates for further reactions, e.g., mutation reactions, PCR reactions, or the like; as substrates for cloning e.g., including digestion or ligation reactions; and/or for identifying exogenous or endogenous modulators of the transcription factors.

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid. A recombinant polynucleotide of the invention can be a cDNA or cDNA-derived polynucleotide that contains the entire coding region of a protein but does not contain the introns of genomic DNA. A recombinant polynucleotide of the invention can also be, or be derived from, a fragment of an isolated genomic DNA that is a full length coding region in that it contains the start of translation of a particular protein through the termination of translation of that same protein, where the start and termination sites are known.

An "isolated polynucleotide" is a polynucleotide or nucleic acid molecule, whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation, but any method can be used as one of skill in the art recognizes.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of the polypeptide. The expression cassette can be introduced into a plant by transformation or by breeding after transformation of a parent plant. A plant refers to a whole plant as well as to a plant part, such as seed, fruit, leaf, or root, plant tissue, plant cell or any other plant material, e.g., a plant explant, as well as to progeny thereof, and to *in vitro* systems that mimic biochemical or cellular components or processes in a cell.

The phrase "ectopic expression or altered expression," or the terms "ectopic expression" or "altered expression" in reference to a polynucleotide or polypeptide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the phrase "ectopic expression or altered expression," or the terms "ectopic expression" or altered expression" may further relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain is a subsequence of the polypeptide that performs at least one biological function of the intact polypeptide in substantially the same manner, or to a similar extent, as does the intact polypeptide. For example, a polypeptide fragment can comprise a recognizable structural motif or functional domain such as a DNA binding site or domain that binds to a DNA promoter region, an activation domain, or a domain for protein-protein interaction. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically of at least about 15 consecutive nucleotides, preferably at least about 30 nucleotides, more preferably at least about 50, of any of the sequences provided herein. A fragment or domain can be referred to as outside a consensus sequence or outside a consensus DNA-binding site that is known to exist or that exists for a particular transcription factor class, family, or sub-family. In this case, the fragment or domain will not include the exact amino acids of a consensus sequence or consensus DNA-binding site of a transcription factor class, family or sub-family, or the exact amino acids of a particular transcription factor consensus sequence or consensus DNA-binding site. Furthermore, a particular fragment, region, or domain of a polypeptide, or a polynucleotide encoding a polypeptide, can be "outside a conserved domain" if all the amino acids of the fragment, region, or domain fall outside of a defined conserved domain(s) for a polypeptide or protein. The conserved domains for polypeptides of the Sequence Listing are listed in the Tables of the Appendices. Also, many of the polypeptides of the Appendices have conserved domains specifically indicated by start and stop sites. A comparison of the regions of the polypeptides in the Sequence Listing, or of those in the Appendices, allows one of skill in the art to identify conserved domain(s) for any of the polypeptides listed or referred to in this disclosure, including those in the Appendices and homologs from other species, strains, or cultivars.

The term "trait" refers to a physiological, morphological, biochemical or physical characteristic of a plant or particular plant material or cell. In some instances, this characteristic is visible to the human eye, such as seed or plant size, or can be measured by biochemical techniques, such as detecting the protein, starch or oil content of seed or leaves, or by the observation of the expression level of a gene or genes, e.g., by employing Northern analysis, RT-PCR, microarray gene expression assays or reporter gene expression systems, or by agricultural observations such as stress tolerance, yield or pathogen tolerance. Any technique can be used to measure the amount of, comparative level of, or difference in any selected chemical compound or macromolecule in the transgenic plants, however.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed (such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### Polnucleotides and Polypeptides of the Invention

The present invention provides, among other things, transcription factors (TFs), and transcription factor homologue polypeptides and homologue polypeptide-encoding polynucleotides (homologs), and isolated or recombinant polynucleotides encoding the polypeptides, or novel variant polypeptides or polynucleotides encoding novel variants of transcription factors derived from the specific sequences provided here. These polypeptides and polynucleotides may be employed to modify one or more of a plant's characteristics or traits.

Exemplary polynucleotides encoding the polypeptides of the invention were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides of the invention were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides of the invention can be or were ectopically expressed in overexpressor or knockout plants and the changes in the characteristic(s) or trait(s) of the plants observed. Therefore, the polynucleotides and polypeptides can be employed to improve the characteristics of plants.

### Producing Polypeptides

The polynucleotides of the invention include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides of the invention. Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed., and 3rd Ed.), Vol. 1-3, Cold Spring Harbor Laboratory, Cold Spring Harbor, New York, ("Sambrook"); Current Protocols in Molecular Biology, F.M. Ausubel et al., eds., Current Protocols, a joint venture between Greene Publishing Associates, Inc. and John Wiley & Sons, Inc., (supplemented through 2001) ("Ausubel"); and Current Protocols in Cell Biology, Bonifacino, J.S. et al. (eds.) 2001 John Wiley & Sons, Inc.

Alternatively, polynucleotides of the invention, can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See*, e.g., Ausubel, Sambrook and Berger, *all supra.*

Alternatively, polynucleotides and oligonucleotides of the invention can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### Homologous Sequences

Sequences homologous, i.e., that share significant sequence identity or similarity, to those provided in the Sequence Listing, derived from *Arabidopsis thaliana* or from other plants of choice are also an aspect of the invention. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus, or mint or other labiates.

Transcription factors that are homologous to the listed sequences will typically share at least about 30% amino acid sequence identity, or at least about 30% amino acid sequence identity outside of a known consensus sequence or consensus DNA-binding site. More closely related transcription factors can share at least about 50%, about 60%, about 65%, about 70%, about 75% or about 80% or about 90% or about 95% or about 98% or more sequence identity with the listed sequences, or with the listed sequences but excluding or outside a known consensus sequence or consensus DNA-binding site, or with the listed sequences excluding or outside one or all conserved domain. Factors that are most closely related to the listed sequences share, e.g., at least about 85%, about 90% or about 95% or more % sequence identity to the listed sequences, or to the listed sequences but excluding or outside a known consensus sequence or consensus DNA-binding site or outside one or all conserved domain. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences, or to a listed sequence but excluding or outside a known consensus sequence or consensus DNA-binding site, or outside one or all conserved domain. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity, and more preferably at least 85%, or at least about 86%, or at least about 87%, or at least about 88%, or at least about 90%, or at least about 95%, or at least about 98% sequence identity. Transcription factors that are homologous to the listed sequences should share at least 30%, or at least about 60%, or at least about 75%, or at least about 80%, or at least about 90%, or at least about 95% amino acid sequence identity over the entire length of the polypeptide or the homolog.

### Identifying Polynucleotides or Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C. For example, high stringency is about 0.2 x SSC, 0.1% SDS at 65° C. Ultra-high stringency will be the same conditions except the wash temperature is raised about 3 or about 5° C, and ultra-ultra-high stringency will be the same conditions except the wash temperature is raised about 6 or about 9°C. For identification of less closely related homologs, washes can be performed at a lower temperature, e.g., 50° C. In general, stringency is increased by raising the wash temperature and/or decreasing the concentration of SSC, as known in the art.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radioactive label, or the like.

Alternatively, transcription factor homolog polypeptides can be obtained by screening an expression library using antibodies specific for one or more transcription factors. With the provision herein of the disclosed transcription factor, and transcription factor homolog nucleic acid sequences, the encoded polypeptide(s) can be expressed and purified in a heterologous expression system (e.g., *E*. *coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### Sequence Variations

It will readily be appreciated by those of skill in the art, that any of a variety of polynucleotide sequences is capable of encoding the transcription factors and transcription factor homologue polypeptides of the invention. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid - serine. Accordingly, at each position in the sequence where there is a codon for serine, any of the above trinucleotide sequences can be used without altering the encoded polypeptide.

**Table 1**

| Amino acid | | | Possible Codons | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide. These conservative variants are, likewise, a feature of the invention.

For example, substitutions, deletions and insertions introduced into the sequences provided in the Sequence Listing are also envisioned by the invention. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### Further Modifying Sequences of the Invention - Mutation/Forced Evolution

In addition to generating silent or conservative substitutions as noted, above, the present invention optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751, and U.S. Patents 5,811,238; 5,811,654; 6,251,604; and 6,177,263. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the invention provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S*. *cerevisiae* and mammals are TAA and TGA, respectively. The preferred stop codon for monocotyledonous plants is TGA, whereas insects and *E. coli* prefer to use TAA as the stop codon.

The polynucleotide sequences of the present invention can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides of the invention can be combined with domains derived from other transcription factors or synthetic domains to modify the biological activity of a transcription factor. For instance, a DNA binding domain derived from a transcription factor of the invention can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### Expression and Modification of Polypeptides

Typically, polynucleotide sequences of the invention are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

The present invention includes recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts that describe molecular biological techniques useful herein, including the use and production of vectors, promoters and many other relevant topics, include Berger, Sambrook and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens*, as well as those disclosed by Herrera-Estrella et al. (1983) Nature 303: 209, Bevan (1984) Nucl Acid Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see*, e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI*, Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present invention also relates to host cells which are transduced with vectors of the invention, and the production of polypeptides of the invention (including fragments thereof) by recombinant techniques. Host cells are genetically engineered (i.e, nucleic acids are introduced, e.g., transduced, transformed or transfected) with the vectors of this invention, which may be, for example, a cloning vector or an expression vector comprising the relevant nucleic acids herein. The vector is optionally a plasmid, a viral particle, a phage, a naked nucleic acid, *etc.* The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be an eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens*, and a portion is stably integrated into the plant genome (Horsch et al. (1984) Science 233:496-498; Fraley et al. (1983) Proc. Natl. Acad. Sci. USA 80, 4803).

The cell can include a nucleic acid of the invention which encodes a polypeptide, wherein the cells expresses a polypeptide of the invention. The cell can also include vector sequences, or the like. Furthermore, cells and transgenic plants, which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

### Modified Amino Acids

Polypeptides of the invention may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### Identification of Additional Factors

A transcription factor provided by the present invention can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor of the invention to modify a phenotype as desired. For example, the transcription factors can be employed to identify one or more downstream gene with which is subject to a regulatory effect of the transcription factor. In one approach, a transcription factor or transcription factor homolog of the invention is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor of the invention can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be performed.

### Identification of Modulators

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998, and supplements through 2001). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and PCT/LTS96/10287), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with the present invention, e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides according to the present invention.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators that inhibit transcriptional activation or transcriptional repression. Either expression of the nucleic acids and proteins herein or any additional nucleic acids or proteins activated by the nucleic acids or proteins herein, or both, can be monitored.

In an embodiment, the invention provides a method for identifying compositions that modulate the activity or expression of a polynucleotide or polypeptide of the invention. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide of the invention.

### Subsequences

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra.*

Subsequences of the polynucleotides of the invention, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologs of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, the invention includes an isolated or recombinant polypeptide including a subsequence of at least about 15 contiguous amino acids encoded by the recombinant or isolated polynucleotides of the invention. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### Production of Transgenic Plants

### Modification of Traits

The polynucleotides of the invention are favorably employed to produce transgenic plants with various traits, or characteristics, that have been modified in a desirable manner, e.g., to improve the seed characteristics of a plant. For example, alteration of expression levels or patterns (e.g., spatial or temporal expression patterns) of one or more of the transcription factors (or transcription factor homologues) of the invention, as compared with the levels of the same protein found in a wild type plant, can be used to modify a plant's traits. An illustrative example of trait modification, improved characteristics, by altering expression levels of a particular transcription factor is described further in the Examples.

### Antisense and Cosuppression Approaches

In addition to expression of the nucleic acids of the invention as gene replacement or plant phenotype modification nucleic acids, the nucleic acids are also useful for sense and anti-sense suppression of expression, e.g., to down-regulate expression of a nucleic acid of the invention, e.g., as a further mechanism for modulating plant phenotype. That is, the nucleic acids of the invention, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens*. After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (*See*, e.g., PCT Publications WO 96/06166 and WO 98/53057, which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides of the invention and/or expressing the polypeptides of the invention can be produced by a variety of well established techniques as described above. Following construction of a vector, most typically an expression cassette, including a polynucleotide, e.g., encoding a transcription factor or transcription factor homologue, of the invention, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al. (1984) Handbook of Plant Cell Culture-Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence into a plant in a manner to cause stable or transient expression of the sequence.

Successful examples of the modification of plant characteristics by transformation with cloned sequences which serve to illustrate the current knowledge in this field of technology, and which are herein incorporated by reference, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide of the invention can be determined by analyzing mRNA expression using Northern blots, RT-PCR or microarrays, or protein expression using immunoblots or Western blots or gel shift assays.

### Integrated Systems - Sequence Identity

Additionally, the present invention may be an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved characteristics, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods for determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill:

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915). Unless otherwise indicated, "sequence identity" here refers to the % sequence identity generated from a tblastx using the NCBI version of the algorithm at the default settings using gapped alignments with the filter "off" (http://www.ncbi.nlm.nih.gov/).

In addition to calculating percent sequence identity, the BLAST algorithm also performs a statistical analysis of the similarity between two sequences (*see*, e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity.

The system may include a link of one or more character strings with a particular phenotype or gene function. Typically, the system includes a user readable output element, which displays an alignment produced by the alignment instruction set.

The methods of this invention can be implemented in a localized or distributed computing environment. In a distributed environment, the methods may be implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intranet or an internet.

Thus, the invention provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### Examples

The following examples are intended to illustrate, but not limit, the scope of the present invention.

### Example I: Full Length Gene Identification and Cloning

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, *Arabidopsis thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60°C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### Example II: Construction of Expression Vectors

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### Example III: Transformation of Agrobacterium with the Expression Vector

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### Example IV: Transformation of Arabidopsis Plants with Agrobacterium tumefaciens with Expression Vector

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### Example V: Identification of Arabidopsis Primary Transformants

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### Example VI: Identification of Arabidopsis Plants with Transcription Factor Gene Knockouts

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### Example VII: Identification of Modified Phenotypes in Overexpression or Gene Knockout Plants

Experiments were performed to identify those transformants or knockouts that exhibited modified biochemical characteristics. Among the biochemicals that were assayed were insoluble sugars, such as arabinose, fucose, galactose, mannose, rhamnose or xylose or the like; prenyl lipids, such as lutein, beta-carotene, xanthophyll-1, xanthophyll-2, chlorophylls A or B, or alpha-, delta- or gamma-tocopherol or the like; fatty acids, such as 16:0 (palmitic acid), 16:1 (palmitoleic acid), 18:0 (stearic acid), 18:1 (oleic acid), 18:2 (linoleic acid), 20:0, 18:3 (linolenic acid), 20:1 (eicosenoic acid), 20:2, 22:1 (erucic acid) or the like; waxes, such as by altering the levels of C29, C31, or C33 alkanes; sterols, such as brassicasterol, campesterol, stigmasterol, sitosterol or stigmastanol or the like, glucosinolates, protein or oil levels.

Fatty acids were measured using two methods depending on whether the tissue was from leaves or seeds. For leaves, lipids were extracted and esterified with hot methanolic H2SO4 and partitioned into hexane from methanolic brine. For seed fatty acids, seeds were pulverized and extracted in methanol:heptane:toluene:2,2-dimethoxypropane:H2SO4 (39:34:20:5:2) for 90 minutes at 80°C. After cooling to room temperature the upper phase, containing the seed fatty acid esters, was subjected to GC analysis. Fatty acid esters from both seed and leaf tissues were analyzed with a Supelco SP-2330 column.

Glucosinolates were purified from seeds or leaves by first heating the tissue at 95°C for 10 minutes. Preheated ethanol:water (50:50) is and after heating at 95°C for a further 10 minutes, the extraction solvent is applied to a DEAE Sephadex column which had been previously equilibrated with 0.5 M pyridine acetate. Desulfoglucosinolates were eluted with 300 ul water and analyzed by reverse phase HPLC monitoring at 226 nm.

For wax alkanes, samples were extracted using an identical method as fatty acids and extracts were analyzed on a HP 5890 GC coupled with a 5973 MSD. Samples were chromatographed on a J&W DB35 mass spectrometer (J&W Scientific).

To measure prenyl lipids levels, seeds or leaves were pulverized with 1 to 2% pyrogallol as an antioxidant. For seeds, extracted samples were filtered and a portion removed for tocopherol and carotenoid/chlorophyll analysis by HPLC. The remaining material was saponified for sterol determination. For leaves, an aliquot was removed and diluted with methanol and chlorophyll A, chlorophyll B, and total carotenoids measured by spectrophotometry by determining absorbance at 665.2 nm, 652.5 nm, and 470 nm. An aliquot was removed for tocopherol and carotenoid/chlorophyll composition by HPLC using a Waters uBondapak C18 column (4.6 mm x 150 mm). The remaining methanolic solution was saponified with 10% KOH at 80°C for one hour. The samples were cooled and diluted with a mixture of methanol and water. A solution of 2% methylene chloride in hexane was mixed in and the samples were centrifuged. The aqueous methanol phase was again re-extracted 2% methylene chloride in hexane and, after centrifugation, the two upper phases were combined and evaporated. 2% methylene chloride in hexane was added to the tubes and the samples were then extracted with one ml of water. The upper phase was removed, dried, and resuspended in 400 ul of 2% methylene chloride in hexane and analyzed by gas chromatography using a 50 m DB-5ms (0.25 mm ID, 0.25 um phase, J&W Scientific).

Insoluble sugar levels were measured by the method essentially described by Reiter et al., Plant Journal 12:335-345. This method analyzes the neutral sugar composition of cell wall polymers found in *Arabidopsis* leaves. Soluble sugars were separated from sugar polymers by extracting leaves with hot 70% ethanol. The remaining residue containing the insoluble polysaccharides was then acid hydrolyzed with allose added as an internal standard. Sugar monomers generated by the hydrolysis were then reduced to the corresponding alditols by treatment with NaBH4, then were acetylated to generate the volatile alditol acetates which were then analyzed by GC-FID. Identity of the peaks was determined by comparing the retention times of known sugars converted to the corresponding alditol acetates with the retention times of peaks from wild-type plant extracts. Alditol acetates were analyzed on a Supelco SP-2330 capillary column (30 m x 250 um x 0.2 um) using a temperature program beginning at 180° C for 2 minutes followed by an increase to 220° C in 4 minutes. After holding at 220° C for 10 minutes, the oven temperature is increased to 240° C in 2 minutes and held at this temperature for 10 minutes and brought back to room temperature.

To identify plants with alterations in total seed oil or protein content, 150mg of seeds from T2 progeny plants were subjected to analysis by Near Infrared Reflectance (NIR) using a Foss NirSystems Model 6500 with a spinning cup transport system.

Experiments were performed to identify those transformants or knockouts that exhibited an improved pathogen tolerance. For such studies, the transformants were exposed to biotropic fungal pathogens, such as *Erisyphe orontii,* and necrotropic fungal pathogens, such as *Fusarium oxysporum. Fusarium oxysporum* isolates cause vascular wilts and damping off of various annual vegetables, perennials and weeds (Mauch-Mani and Slusarenko (1994) Molecular Plant-Microbe Interactions 7: 378-383). For *Fusarium oxysporum* experiments, plants grown on petri dishes were sprayed with a fresh spore suspension of *F. oxysporum.* The spore suspension was prepared as follows: A plug of fungal hyphae from a plate culture was placed on a fresh potato dextrose agar plate and allowed to spread for one week. 5 ml sterile water was then added to the plate, swirled, and pipetted into 50 ml Armstrong Fusarium medium. Spores were grown overnight in Fusarium medium and then sprayed onto plants using a Preval paint sprayer. Plant tissue was harvested and frozen in liquid nitrogen 48 hours post infection.

*Erysiphe orontii* is a causal agent of powdery mildew. For *Erysiphe orontii* experiments, plants were grown approximately 4 weeks in a greenhouse under 12 hour light (20 C, ~30% relative humidity (rh)). Individual leaves were infected with *E. orontii* spores from infected plants using a camel's hair brush, and the plants were transferred to a Percival growth chamber (20 C, 80% rh.). Plant tissue was harvested and frozen in liquid nitrogen 7 days post infection.

Botrytis cinerea is a necrotrophic pathogen. Botrytis cinerea was grown on potato dextrose agar in the light. A spore culture was made by spreading 10 ml of sterile water on the fungus plate, swirling and transferring spores to 10 ml of sterile water. The spore inoculum (approx. 105 spores/ml) was used to spray 10 day-old seedlings grown under sterile conditions on MS (-sucrose) media. Symptoms were evaluated every day up to approximately 1 week.

Infection with bacterial pathogens Pseudomonas syringae pv maculicola strain 4326 and pv maculicola strain 4326 was performed by hand inoculation at two doses. Two inoculation doses allows the differentiation between plants with enhanced susceptibility and plants with enhanced resistance to the pathogen. Plants were grown for 3 weeks in the greenhouse, then transferred to the growth chamber for the remainder of their growth. Psm ES4326 was hand inoculated with 1 ml syringe on 3 fully-expanded leaves per plant (4 1/2 wk old), using at least 9 plants per overexpressing line at two inoculation doses, OD=0.005 and OD=0.0005. Disease scoring occured at day 3 post-inoculation with pictures of the plants and leaves taken in parallel.

In some instances, expression patterns of the pathogen-induced genes (such as defense genes) was monitored by microarray experiments. cDNAs were generated by PCR and resuspended at a final concentration of ~ 100 ng/ul in 3X SSC or 150mM Na-phosphate (Eisen and Brown (1999) Meth. in Enzymol. 303:179-205). The cDNAs were spotted on microscope glass slides coated with polylysine. The prepared cDNAs were aliquoted into 384 well plates and spotted on the slides using an x-y-z gantry (OmniGrid) purchased from GeneMachines (Menlo Park, CA) outfitted with quill type pins purchased from Telechem International (Sunnyvale, CA). After spotting, the arrays were cured for a minimum of one week at room temperature, rehydrated and blocked following the protocol recommended by Eisen and Brown (1999).

Sample total RNA (10 ug) samples were labeled using fluorescent Cy3 and Cy5 dyes. Labeled samples were resuspended in 4X SSC/0.03% SDS/4 ug salmon sperm DNA/2 ug tRNA/ 50mM Na-pyrophosphate, heated for 95°C for 2.5 minutes, spun down and placed on the array. The array was then covered with a glass coverslip and placed in a sealed chamber. The chamber was then kept in a water bath at 62°C overnight. The arrays were washed as described in Eisen and Brown (1999) and scanned on a General Scanning 3000 laser scanner. The resulting files are subsequently quantified using Imagene software purchased from BioDiscovery (Los Angeles, CA).

Experiments were performed to identify those transformants or knockouts that exhibited an improved environmental stress tolerance. For such studies, the transformants were exposed to a variety of environmental stresses. Plants were exposed to chilling stress (6 hour exposure to 4-8° C), heat stress (6 hour exposure to 32-37°C), high salt stress (6 hour exposure to 200 mM NaCl), drought stress (168 hours after removing water from trays), osmotic stress (6 hour exposure to 3 M mannitol), or nutrient limitation (nitrogen, phosphate, and potassium) (Nitrogen: all components of MS medium remained constant except N was reduced to 20mg/L of NH₄ NO₃, or Phosphate: All components of MS medium except KH₂ PO₄, which was replaced by K₂SO₄, Potassium: All components of MS medium except removal of KNO₃ and KH₂PO₄, which were replaced by NaH₄PO₄).

Experiments were performed to identify those transformants or knockouts that exhibited a modified structure and development characteristics. For such studies, the transformants were observed by eye to identify novel structural or developmental characteristics associated with the ectopic expression of the polynucleotides or polypeptides of the invention.

Experiments were performed to identify those transformants or knockouts that exhibited modified sugar-sensing. For such studies, seeds from transformants were germinated on media containing 5% glucose or 9.4% sucrose which normally partially restrict hypocotyl elongation. Plants with altered sugar sensing may have either longer or shorter hypocotyls than normal plants when grown on this media. Additionally, other plant traits may be varied such as root mass.

Flowering time was measured by the number of rosette leaves present when a visible inflorescence of approximately 3 cm is apparent. Rosette and total leaf number on the progeny stem are tightly correlated with the timing of flowering (Koornneef et al (1991) Mol. Gen. Gene 229:57-66). The vernalization response was measured. For vernalization treatments, seeds were sown to MS agar plates, sealed with micropore tape, and placed in a 4°C cold room with low light levels for 6-8 weeks. The plates were then transferred to the growth rooms alongside plates containing freshly sown non-vernalized controls. Rosette leaves were counted when a visible inflorescence of approximately 3 cm was apparent.

Modified phenotypes observed for particular overexpressor or knockout plants are provided in Table 4 of the Appendix and the Appendices of the priority documents. For a particular overexpressor that shows a less beneficial characteristic, it may be more useful to select a plant with a decreased expression of the particular transcription factor. For a particular knockout that shows a less beneficial characteristic, it may be more useful to select a plant with an increased expression of the particular transcription factor.

The sequences of the Sequence Listing SEQ ID Nos. 1-464 or those disclosed here can be used to prepare transgenic plants and plants with altered traits. The specific transgenic plants listed below are produced from the sequences of the Sequence Listing, as noted. The Tables of the Appendix and the Appendices of the priority documents provide exemplary polynucleotide (cDNA) and polypeptide (protein) sequences of the invention. The Tables includeSEQ ID Nos., the corresponding reference number (GID), and/or the identification of the start and stop residues of any conserved domain in the polypeptide sequence.

The transgenic plants of the invention display an ectopic expression or altered expression of one or more polypeptides encoded by the full length coding regions in the Sequence Listing, the homologs and/or fragments of the Tables of the Appendices, and/or another polypeptide described in this document, when the transgenic plant is compared to a wild type, control, or reference plant. As a result, the transgenic plants possess advantageous traits, as detailed by the limited and exemplary discussion of comparison data below.

Some of the polypeptides encoded by the full length coding regions in the Sequence Listing and the homologs and fragments of them noted in the Tables of the Appendices modulate a plant's defense response and even confer multipathogen resistance. These traits are extremely useful in many commercial crops and plants. For example, plants overexpressing G28 (SEQ ID NO.: 1 and 2) are more tolerant to infection by fungal pathogens, such as Erysiphe orontii, Sclerotinia sclerotiorum, or Botrytis cinerea. Similarly, plants overexpressing G1792 (SEQ ID NO.: 5 and 6) are more tolerant to infection by necrotrophic fungal pathogens, such as Fusarium oxysporum or Botrytis cinerea, and display increased resistance to fungal pathogens and to Erysiphe orontii. Increased tolerance to infection by Fusarium oxysporum is observed in G1047 (SEQ ID NO.: 23 and 24) and G1363 (SEQ ID NO.: 29 and 30) overexpressing plants. Knockout mutants also demonstrate the particular polypeptide's involvement in a defense response. A G1880 (SEQ ID NO.: 435 and 436) knockout mutant is more tolerant to Botrytis cinerea. G1196 (SEQ ID NO.: 27 and 28) knockout mutant plants show increased susceptibility to Botrytis cinerea. Manipulating the content or expression of any of these polypeptides, or fragments or homologs of them, can therefore improve a plant's defense response, tolerance, or susceptibility to pathogens and infection.

A number of the polypeptides encoded by the full length coding regions in the Sequence Listing, and homologs and fragments of them noted in the Tables of the Appendices, regulate the transition from vegetative to reproductive growth. These traits can be useful in crops and plants where fruit or seed is commercially valuable, for example. Overexpression of G180 (SEQ ID NO.: 53 and 54), G227 (SEQ ID NO.: 313 and 314), G1841 (SEQ ID NO.: 455 and 456), and G2347 (SEQ ID NO.: 477 and 478) results in an early flowering phenotype, whereas overexpression of G748 (SEQ ID NO.: 125 and 126) or G2007 (SEQ ID NO.: 457 and 458) results in late flowering. Other polypeptides and polynucleotides for modulating flowering time include G590 (SEQ ID NO.: 107 and 108), G1760 (SEQ ID NO.: 31 and 32), G1820 (SEQ ID NO.: 33 and 34), and G2010 (SEQ ID NO.: 37 and 38).

The response to a variety of abiotic or environmental stresses is modified by an additional set of polypeptides encoded by the full length coding regions of the Sequence Listing and the homologs and fragments listed in the Tables of the Appendices. These traits can be useful in manipulating the growth medium or environment for plants, for example. G226 overexpressing plants are more tolerant to low nitrogen and high salt stress. G2130 (SEQ ID NO.: 417 and 418) overexpressors show improved heat stress tolerance in a germination assay. G867 (SEQ ID NO.: 15 and 16) and G1930 (SEQ ID NO.: 35 and 36) overexpressing plants show increased seedling vigor in germination assays on both high salt and high sucrose containing media. G912 (SEQ ID NO.: 19 and 20) is a member of the AP2 family related to the CBF1, CBF2 and CBF3 genes. Plants overexpressing G912 (SEQ ID NO.: 19 and 20) exhibit increased freezing and drought tolerance. Additional polypeptides and polynucleotides modifying stress responses include G175 (SEQ ID NO.: 9 and 10), G926 (SEQ ID NO,: 459 and 460), and G1820 (SEQ ID NO.: 33 and 34).

Several transcription factors have been identified that can affect metabolic processes. These plants can be used to optimize or improve production of various plants extracts used for commercial products including, for example, foodstuffs, paper and paper-related products, edible plants, fruits and vegetables with improved properties, organic compounds, oils and alcohols, additives and binders for pharmaceutical or cosmetic products, and industrial products. For instance, plants overexpressing G1750 (SEQ ID NO.: 395 and 396) produce seed with increased seed oil content. Overexpression of G280 (SEQ ID NO.: 461 and 462) results in an increase in gamma and delta tocopherol in leaves. G663 (SEQ ID NO.: 13 and 14) overexpressors exhibit constitutive anthocyanin production in seeds, leaves and roots. In contrast, seeds of G156 (SEQ ID NO.: 7 and 8) knockout mutant plants exhibit a colorless phenotype indicative of the involvement of the gene in the regulation of the anthocyanin pathway.

Also of particular interest are polypeptides involved in plant growth and development. The following polypeptides encoded by the full length coding regions of the Sequence Listing and the homologs and fragments listed in the Tables of the Appendices are some examples. Transgenic plants overexpressing G1073 exhibit a substantial increase in size. An increase in size is also observed in G189 (SEQ ID NO.: 11 and 12) overexpressing plants. Transgenic plants overexpressing G634 (SEQ ID NO.: 3 and 4) exhibit a substantial increase in trichome number. Null mutations in G374 (SEQ ID NO.: 463 and 464) and in G877 (SEQ ID NO.: 17 and 18) result in embryo lethality. A G979 (SEQ ID NO.: 153 and 154) knockout mutation results in delayed seed ripening.

G987 (SEQ ID NO.: 21 and 22) knockout mutant plants can only be grown on sucrose-containing medium. In addition, G987 appears to control an aspect of thylakoid membrane development and the tocopherol, carotenoid, and/or chlorophyll content of the plant is altered. Since the compounds represented by these groups are commercially important in a number of industries, including use as dietary supplements, a transgenic plant's altered tocopherol, carotenoid, and/or chlorophyll content is an advantageous and valuable trait.

### G634 (SEQ ID. Nos 3 and 4), G1841 (SEQ ID. Nos 455 and 456), G979 (SEQ ID. Nos 153 and 154): modified plant development

**G634:** Overexpression of G634 produced an increase in trichome density on later arising rosette leaves, cauline leaves, inflorescence stems and sepals. Trichomes of 35S::G634 plants also appeared slightly larger than those of wild type, and stem trichomes were more highly branched. These effects were not apparent in young seedlings and became most prominent at the later vegetative and early reproductive phase. The trichome phenotype was apparent in approximately 50% of primary transformants and two out of the three T2 lines.

**G1841:** Overexpression of G1841 markedly reduced the time to flowering. This early flowering phenotype was consistently observed over multiple plantings for each of the three T2 lines, and in a majority of primary transformants. Additionally, 35S::G1841 plants appeared slightly pale and had rather flat leaves compared to wild-type controls.

In continuous light conditions, 35S::G1841 plants produced flower buds up to five days earlier than wild-type controls. In repeat sowings the plants appeared to grow slightly faster than controls; although they switched to making flower buds several days early, they had a similar number of primary rosette leaves to wild type.

In addition to showing accelerated flowering under 24 hours light, plants from all three T2 populations produced flowers up to 2 weeks earlier than controls under a 12 hour photoperiod.

**G979:** Seeds homozygous for a T-DNA insertion within G979 showed delayed ripening, slow germination, and developed into small, poorly fertile plants, indicating that G979 might be involved in seed development processes.

Siliques of heterozygous plants were examined for seed abnormalities. Approximately 25% of the seeds contained in young green siliques were pale in coloration. In older, brown siliques, approximately 25% of the seeds were green and appeared slow ripening, whereas the remaining seeds were brown. Thus, it seemed likely that the seeds with altered development were homozygous for the T-DNA insertion, whereas the normal seeds were wild type and heterozygous segregants.

Furthermore, it was observed that approximately 25% of the seed from G979 KO heterozygous plants showed impaired (delayed) germination. Upon germination, these seeds produced extremely tiny seedlings that often did not survive transplantation. A few homozygous plants, small and sickly looking, could be grown, and produced siliques that contained seeds that were small and wrinkled compared to wild type.

On the basis of these results obtained with G979 knockout mutant lines, G979 can be used to alter or modify seed germination properties and performance.

### G1792 (SEQ ID. Nos 5 and 6), G2130 (SEQ ID. Nos 417 and 418), G926 (SEQ ID. Nos. 459 and 460): modified stress response

**G1792:** 35S::G1792 plants were more tolerant to the fungal pathogens *Fusarium oxysporum* and *Botrytis cinerea:* they showed fewer symptoms after inoculation with a low dose of each pathogen. This result was confirmed using individual T2 lines.

35S::G1792 plants also showed more tolerance to growth under nitrogen-limiting conditions. In a root growth assay under conditions of limiting N, 35S::G1792 lines were slightly less stunted. In a germination assay that monitors the effect of C on N signaling through anthocyanin production on high sucrose plus and minus, the 35S::G1792 lines make less anthocyanin on high sucrose plus glutamine, suggesting that the gene could be involved in the plants ability to monitor their carbon and nitrogen status.

G1792 overexpressing plants also showed several mild morphological alterations such as abnormal phyllotaxy, alterations in leaf and flower development, and flowering time.

**G2130:** G2130 overexpressing lines show more seedling vigor in a heat stress tolerance germination assay compared to wild-type controls. No difference from wild-type was detected in the heat stress response assay performed on older seedlings suggesting the phenotype could be specific for germination in the G2130 overexpressors. Lines G2130-3 and G2130-4 show the heat tolerant phenotype, line G2130-2 show the weakest phenotype. G2130 overexpressing lines are also somewhat more sensitive to chilling, the plants are more chlorotic and stunted when grown at 8oC compared to the wild-type controls. They also show more disease symptoms following inoculation with a low dose of the fungal pathogen *Botrytis cinerea* in two separate experiments.

**G926:** G926 knockout mutant plants show more tolerance to osmotic stress in a germination assay in three separate experiments. They show more seedling vigor than wild-type controls when germinated on plates containing high salt and high sucrose. They also show insensitivity to ABA in repeated germination assays.

These analyses revealed that in the absence of G926 function, plants are more tolerant to osmotic stress. This osmotic stress tolerance could be related to the plant's apparent insensitivity to the growth hormone ABA because ABA plays an important regulatory role in the initiation and maintenance of seed dormancy. G926 may function as part of a checkpoint for germinating seeds and loss of G926 function promotes germination regardless of the osmotic status of the environment. G926 has utility in modifying plant stress responses.

### G280 (SEQ ID. Nos. 461 and 462), G1323 (SEQ ID. Nos 203 and 204): modified biochemistry

**G280:** Overexpression of G280 in Arabidopsis resulted in an increase in leaf gamma and delta tocopherol in all three lines tested. Overexpression of G280 produced a reduction in overall plant size and accelerated the rate of leaf senescence in the rosette.

**G1323:** In two G1323 overexpressing lines, line 5 and 7, seeds had more protein and less oil than controls. Otherwise, overexpression of G1323 in Arabidopsis did not result in any biochemical phenotype. These experiments were repeated and a similar biochemical phenotype was observed.

### G2557 (SEQ ID Nos. 289 and 290), G2143 (SEQ ID Nos. 285 and 286), G1063 (SEQ ID Nos 167 and 168) (HLH/MYC)

Overexpression of each of these genes affected plant growth, inflorescence architecture, and resulted in the development of carpelloid tissues in ectopic positions.

G2557: Twenty independent 35S::G2557 *Arabidopsis* primary transformants were obtained. Of these plants, 19/20 exhibited carpelloid tissue in the outer whorl organs of flowers. In some instances ovules developed from these ectopic carpels. The central carpel of 35S::G2557 flowers was also sometimes borne on a long pedicel-like structure, indicating that overexpression of this gene could influence determinacy of the floral meristem. Additionally, 35S::G2557 plants were often smaller, darker green and possessed narrow leaves and elongated cotyledons compared to wild type.

G2143: Twenty independent 35S::G2143 *Arabidopsis* primary transformants were obtained. All 20 plants developed ectopic carpelloid tissue. In some cases entire flowers were replaced by a disorganized mass of this tissue. Additionally, 35S::G2143 plants were often smaller, darker green and possessed narrow leaves and elongated cotyledons compared to wild type. In some cases the shoot tips of G2413 plants aborted in a 'pin-like' structure.

G1063: Seventeen independent 35S::G1063 *Arabidopsis* primary transformants were obtained. 5/17 of these lines exhibited flowers in which outer whorl organs displayed carpelloid features. In some cases flowers were completely replaced by a carpelloid mass of tissue and defined individual organs could not be distinguished. The shoots of these plants also occasionally terminated in a 'pin-like' structure. The majority of 35S::G1063 plants were smaller than wild type and often had altered leaf shape.

Based on the above phenotypes, these genes might be applied to manipulate flower structure and development, fertility, seed-pod development, leaf coloration and overall plant architecture. Specifically, the genes might be used to manipulate floral organ identity or instigate the formation of carpel-derived structures including ovules, embryos and seeds.

### G2509 (SEQ ID Nos 287 and 288) (AP2)

Twenty independent 35S::G2509 *Arabidopsis* primary transformants were obtained. All plants exhibited increased secondary shoot development and loss of apical dominance, leading to a shorter bushier stature than wild type. G2509 could be used to modify plant architecture. This could produce plants more resistant to wind and rain and influence yield. Additionally, changing plant architecture could generate novel interesting forms for the ornamental plant market.

### G353 (SEQ ID Nos 79 and 80) and G354 (SEQ ID Nos. 81 and 82) (Z(C2H2))

G353 and G354 constitute a pair of closely related Z(C2H2) genes that influence shoot architecture. Both genes produced comparable effects when overexpressed.

G353: A consistent phenotype was noted on inflorescences of 35S::G353 plants. Flowers were oriented downwards and pedicels of flowers and siliques were reduced in length or absent. Floral internodes were also very short. Furthermore, secondary shoots were often observed to grow in a downward direction. These phenotypes were observed in both primary transformants and T2 generation plants. Overexpression of G353 produced additional effects; 35S::G353 were sometimes smaller than wild-type, had abnormal branching patterns and flat leaves.

G354: 35S::G354 plants displayed abnormal inflorescences in which flowers were oriented downwards and pedicels were absent or reduced in length. Floral internodes were also short. Additionally, many of the 35S::G354 plants were reduced in size compared to wild type.

These genes could be used to modify plant architecture. Specifically, altering the length of flower and fruit stalks could permit more efficient harvesting. In species such as strawberry, changing the length of the fruit stalk could allow fruits to develop above the leaf canopy and reduce the likelihood of fungal infection. The genes might also have applications in producing novel forms of ornamental species in which branches, flowers and fruits develop with unusual orientations.

### G1494 (SEQ ID Nos. 223 and 224) (HLH/MYC)

The phenotype of transgenic *Arabidopsis,* over-expressing G1494, indicates that this gene is a core component of the plant light perception/response machinery. 35S::G1494 seedlings displayed very long hypocotyls, bolted early, and exhibited elongation of rosette internodes. This latter characteristic resulted in the absence of a defined rosette. The plants also possessed very spindly stems, and narrow pale leaves with elongated petioles. Such features were consistently observed in both primary transformants and T2 generation plants. These phenotypes are comparable to those of mutants defective in the *PHYTOCHROME* genes, which encode proteins involved in the perception of light conditions. In particular, the 35S::G1494 phenotype is almost identical to that described for the *phyA;phyB;phyD* triple mutant (Devlin et al., Plant Physiology 119, 909-915). Based upon the 35S::G1494 phenotype, this gene might be applied to manipulate many of the traits which are influenced by the perception and response to light, including seed germination, flowering time, shade response, leaf orientation, architecture and growth habit.

Additional phenotypes that were observed included G634 (SEQ ID Nos. 3 and 4) (overexpressors had substantially more trichomes on its leaf surfaces), G971 (SEQ ID Nos. 17 and 18) (overexpressors enhanced terpenoid biosynthesis levels) and G1792 (SEQ ID Nos. 5 and 6) (overexpressors showed a broad-based disease resistance).

### Example VIII: Identification of Homologous Sequences

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919).

Identified *Arabidopsis* homologous sequences are provided in the Tables of the Appendices. The percent sequence identity among these sequences can be as low as 47%, or even 31% or lower sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*). These sequences are compared to sequences representing genes of SEQ IDs Nos. 1-16 using the Washington University TBLASTX algorithm (version 2.0a19MP) at the default settings using gapped alignments with the filter "off,"as performed on July 16, 2001 or previously. For each gene of the Sequence Listing, individual comparisons were ordered by probability score (P-value), where the score reflects the probability that a particular alignment occurred by chance. For example, a score of 3.6e-40 is 3.6 x 10⁻⁴⁰. In addition to P-values, comparisons were also scored by percentage identity. Percentage identity reflects the degree to which two segments of DNA or protein are identical over a particular length.

In addition to computer-based methods for identifying homologs, or indeed in conjunction with them, a fragment of a sequence from the sequence listing, from the Tables of the Appendices, or derived from a homolog sequence identified from a database, is radiolabeled with ³²P by random priming (Sambrook et al., Molecular Cloning. A Laboratory, Manual, 2nd Ed., or 3rd Ed., Cold Spring Harbor Laboratory Press, New York) and used to screen a plant cDNA or genomic library. As merely one example, total plant DNA from *Arabidopsis thaliana, Nicotiana tabacum, Lycopersicon pimpinellifolium, Prunes avium, Prunus cerasus, Cucumis sativus,* or *Oryza sativa* is isolated (Stockinger, E.J., et al., (1996), J. Heredity, 87:214-218). Alternatively, cDNA clones of a selected cDNA library are used. Approximately 2 to 10 µg of each DNA sample is restriction digested, transferred to nylon membrane (Micron Separations, Westboro, MA) and hybridized. Alternatively, a library is plated out on growth medium and partially transferred *in situ* to the nylon membrane for hybridization. Exemplary hybridization conditions are: 42°C in 50% formamide, 5X SSC, 20 mM phosphate buffer, 1X Denhardt's, 10% dextran sulfate, and 100 µg/ml herring sperm DNA. Four low stringency washes at RT in 2X SSC, 0.05% sodium sarcosyl and 0.02% sodium pyrophosphate are performed prior to high stringency washes at 55°C in 0.2X SSC, 0.05% sodium sarcosyl and 0.01% sodium pyrophosphate. High stringency washes are performed until no counts are detected in the washout (Walling, L.L., et al., Nucl. Acids Res. 16:10477-10492(1988)). The areas of radioactivity on the membrane correspond to homologous sequences from the library or genomic DNA sample and the associated DNA can be identified, isolated, and cloned into an appropriate vector so that any homologous sequence(s) can be used. Alterations in the stringency of washes, such as employing ultra-high stringency, and ultra-ultra-high stringency, can also be made.

### Example IX

As noted previously, the introduction of polynucleotides of the invention and full length coding sequences of the invention into the target plant or cell can be accomplished by a variety of techniques known in the art, such as calcium phosphate-DNA precipitation, electroporation, microinjection, Agrobacterium infection, liposomes, or microprojectile bombardment, for example. Those of ordinary skill in the art can refer to the literature for details and select suitable techniques without undue experimentation. For some plants, using Agrobacterium is a preferred and easy method for transforming plants and cells. This type of transformation has been used for genetic manipulation of more than 120 species of at least 35 different families of plants, including major economic crops such as vegetables, ornamentals, medicinals, fruit, trees and pasture plants (see, for example, Birch, R.G., Annual Rev. Plant Physiology and Plant Molec. Biology 48:297-326 (1997); Gould J.H., Transformation of the Cereals using Agrobacterium, In: R.S. Tuan (Ed.), Methods in Molecular Biology, Humana Press Inc., Totowa, NJ, vol. 62:489-499 (1997)). In fact, this method has become so routine and commonplace that the idea that some species cannot accept the integration of foreign DNA into its genome or that a species lacks the capacity to be transformed has become unacceptable in the art (see de la Riva et al., Electr. J. Biotechnol. Agrobacterium tumefaciens: a natural tool for plant transformation, vol. 1, no. 13, issue of Dec. 15, 1998).

A number of vectors can be used to produce transgenic plants. Some of these vectors can replicate in bacterial hosts, plant host cells, and Agrobacterium, as known through many techniques of the art. Expression vectors typically comprise a cassette or region for inserting a coding sequence or transgene that is flanked by a promoter/enhancer and a poly A site. Many variations are possible, including the use of sequences incorporating preferred codons, 5' UTR, 3' UTR, splice donor and acceptor or other intron sequences, internal ribosome entry sites, repressor or suppressor binding sequences, tissue-specific promoters and enhancers, developmentally regulated promoters and enhancers, and inducible promoters and enhancers, for example. Examples of inducible promoters useful in plants include those induced by chemical means, such as the yeast metallothionein promoter, which is activated by concentrations of copper or heavy metal ions. Any appropriate inducible promoter, enhancer, or promoter/enhancer can be selected. One skilled in the art can devise many variations and permutations in selecting and using expression vectors. The vectors may also contain selectable markers for more easily identifying transformed plants. Many types of selectable marker genes are known in the art.

If using Agrobacterium, one can select armed or disarmed Ti genes for transforming cells and plants. Either Ti plasmids of Agrobacterium tumefaciens (A. tumefaciens) or root-inducing (Ri) plasmids of Agrobacterium rhizogenes (A. rhizogenes) can be selected. (For reviews of exemplary techniques see, for example, Weissbach & Weissbach, (1988) Methods for Plant Molecular Biology, Academic Press, NY, Section VIII, pp. 421-463; and Grierson & Corey (1988) Plant Molecular Biology, 2d Ed., Blackie, London, Ch. 7-9, and Horsch et al., Science 227:1229 (1985), incorporated herein by reference). The selection of either A. tumefaciens or A. rhizogenes will depend on the plant being transformed. In general A. tumefaciens is the preferred organism for transformation. Most dicotyledons, some gymnosperms, and a few monocotyledons (e.g. certain members of the Liliales and Arales) are easily susceptible to infection with A. tumefaciens. A. rhizogenes also has a wide host range, including most dicots and gymnosperms, which includes members of the Leguminosae, Compositae and Chenopodiaceae. Selecting a type of vector and the components of the vector is well within the ordinary skill of the art.

A general and exemplary method for plant transformation with Agrobacterium follows. The polynucleotide or the full length coding region (transgene) is inserted into an intermediate or shuttle vector capable of replicating in E. coli and suitable for the type of plant used and typically containing a selectable marker. The vector is introduced into an acceptor A. tumefaciens strain through triparental mating (reciprocal recombination between the intermediate vector and the T-DNA region of the acceptor plasmid occurs during triparental mating and the transgene is now part of the T-DNA region that will be transferred). The engineered A. tumefaciens strain containing the transgene is cocultivated with a plant explant, from which regenerated plants can be obtained. The explants are cultured in the presence of a selection agent and selecting resistant cells grow shoots and rooted-shoots. These are regenerated into plants and the regenerated plants screened for the expression of the transgene and selectable marker. The progeny of the transgenic plant is grown and the inheritance of the introduced transgene is determined.

A transgenic plant transformed using Agrobacterium typically contains a single copy of the introduced transgene on one chromosome - it is heterozygous for the transgene. Homozygous plants can also be prepared and can be preferred or more stable in certain plants. One skilled in the art is familiar with breeding and crossing techniques to produce homozygous plants regardless of the type of transformation used. For example, homozygous transgenic plants can be produced through sexually mating an independent segregant that contains a single transgene, germinating the seed of the plant, and selecting the plants produced for the transgene. In addition, two transformed or transgenic plants can be mated to produce plants having two independently segregating transgenes. Sexually mating progeny produces homozygous plants for both transgenes. Those of skill in the art are also familiar with techniques, such as back-crossing to parental plants, out-crossing with a wild type or non-transgenic plant, and vegetative propagation, for example, to manipulate plants having one or more transgenes. Any of these techniques can be employed to produce transgenic plants, seeds, plant cells, or plant tissue or extracts having a polynucleotide or polypeptide of the invention.

Another common transformation protocol employs plant protoplasts using methods based on calcium phosphate precipitation, polyethylene glycol treatment, electroporation, and combinations of these. The selection of a protoplast method depends upon the ability to regenerate that particular plant strain from protoplasts. Many methods for regenerating plants from protoplasts exist and any can be selected for use (see, for example Shillito, R.D. and Saul, M.W., Protoplast Isolation and Transformation, In: Plant molecular biology, A Practical Approach, IRL Press, UK (1988), particularly pp. 161-186; Methods in Enzymology, vol. 118, (Plant Molecular Biology), eds. Weissbach, A. and Weissbach, H., Academic Press, Orlando, Florida (1985); Methods in Enzymology, vol. 153 (Recombinant DNA), eds. Wu, R. and Grossman, L., Academic Press, Orlando, Florida, (1987).

To transform plant strains that cannot be successfully regenerated from protoplasts, other ways to introduce DNA into intact cells or tissues can be utilized. For example, plants can be regenerated from immature embryos or explants following introduction of vector or expression cassette DNA containing the transgene. The methods used to regenerate transformed cells into whole plants are not critical to this invention and any method suitable for the target plant can be employed. The literature describes numerous techniques for regenerating specific plant types (for example, somatic embryogenesis, Umbeck, P., et al., Genetically transformed cotton (Gossypium hirsutum L.) plants, Bio/Technology 5:263 266 (1987)), and other techniques are continually becoming known. One of ordinary skill in the art can refer to the literature for details and select suitable techniques without undue experimentation. In practice, a large number of transformed plants can be routinely regenerated from a transformed plant cell or tissue to increase and maintain a sterile line. Many methods for culturing plant cells and regenerating transformed plants from cells are known in the art and any appropriate method can be selected (see, for example, Plant Tissue and Cell Culture, C. E. Green, D. A. et al., (Eds.), Alan R. Liss, Inc., New York; Experiments in Plant Tissue Culture, Dodds, J. H. et al. (Eds.), 1985, Cambridge University Press; Cell Structure and Somatic Cell Genetics of Plants, Vasil, I. K. (Ed.), 1984, Academic Press; Handbook of Plant Cell Culture, Volume 4, Techniques and Applications, Evans, D.A. et al. (Eds.), 1986, Macmillan Publishing Company).

In addition, microprojectile bombardment techniques can be used and many have been described in the art. Here, DNA is carried through the cell wall and into the cytoplasm on the surface of small metal particles (see, for example McCabe et al., Bio/Technology 6:923 (1988)). The metal particles penetrate through several layers of cells and allow the transformation of cells within tissue explants. These explants or cells of them can then be regenerated into plants.

For example, if soybean is selected, the following method can be used. Somatic embryos, cotyledons, 3-5 mm in length, are dissected from surface of sterilized, immature seeds of the soybean cultivar chosen, and the embryos cultured in light or darkness at 26°C on an appropriate agar medium for 6-10 weeks. Somatic embryos that produce secondary embryos are then excised and placed into a suitable liquid medium. After repeated selection for clusters of somatic embryos that multiply, the suspensions are maintained in suspension culture.

The soybean embryogenic suspension cultures can maintained in 35 ml liquid media on a rotary shaker, 150 rpm, at 26°C with florescent lighting on a 16:8 hour day/night schedule. Subculturing every two weeks by inoculating approximately 35 mg of tissue into 35 ml of liquid medium maintains the cells.

A DuPont BioliStic PDS1000/HE instrument, a BIO RAD PDS-1000/He or other microprojectile device can be used for these transformations. DNA-coated microcarriers, typically tungsten or gold microparticles, are used according to the instruction manual. To 50 µl of a 60 mg/ml 1 µm gold particle suspension is added 5 µl DNA (1 µg/µl), 20 µl spermidine (0.1 M), and 50 µl CaCl2 (2.5 M). The particle preparation is agitated for three minutes, spun in a microfuge for 10 seconds, and the supernatant is removed. The DNA-coated particles are then washed once in 400 µl 70% ethanol and resuspended in 40 µl of anhydrous ethanol. The DNA/particle suspension can be sonicated three times for one second each. Five µl of the DNA-coated gold particles is loaded on the disk or appropriate carrier for the particle gun.

Approximately 300-400 mg of a two-week-old suspension culture is placed in an empty petri dish and the residual liquid removed from the tissue with a pipette. For each transformation, approximately 5-10 plates of tissue are normally used. Membrane rupture pressure is set at approximately 1100 psi. The tissue is placed approximately 3.5 inches away from the retaining screen and bombarded three times. Following treatment, the tissue can be divided in half and placed back into liquid and cultured as above.

Five to seven days post bombardment, the liquid media is exchanged with fresh media, and eleven to twelve days post bombardment with fresh media containing selection media (if the vector or DNA used also encodes a selectable marker, as it preferably will). The selection media is replaced approximately ever week. Seven to eight weeks post bombardment, green, transformed tissue may be observed growing from un-transformed, necrotic embryogenic clusters. Isolated green tissue is removed and inoculated into individual flasks to generate new, clonally propagated, transformed embryogenic suspension cultures. Each new line may be treated independently. These suspensions can then be sub-cultured and maintained as clusters of immature embryos or regenerated into whole plants by maturation and germination of individual somatic embryos.

If maize is selected, immature embryos are excised from cleaned and sterilized ears and placed embryo axis side down (scutellum side up) in a petri plate. These are cultured in 560L medium for 4 days in the dark. To prepare for bombardment, the embryos are transferred to 560Y medium for 4 hours and arranged within the device target zone.

The DNA is prepared with Tungsten microparticles, for example, using 1 ug DNA in Tris EDTA buffer, 2.5 M CaCl2, and 0.1 M spermidine while vortexing. The mixture is sonicated briefly and incubated under constant vortexing for ten minutes. After a precipitation period, the tubes are centrifuged briefly, and the liquid is removed. The particles are washed with 100% ethanol, centrifuged, and resuspended in 100% ethanol. For particle gun bombardment, the tungsten/DNA particles are briefly sonicated and 10 ul spotted onto the center of each carrier and allowed to dry about 2 minutes before bombardment.

All samples receive a single shot at approximately 650 psi. Following bombardment, the embryos are cultured in 560Y medium for 2 days then transferred to 560R selection medium and sub-cultured every 2 weeks. After approximately 10 weeks of selection, selection-resistant callus clones are sampled by PCR for transgene content and/or activity analysis. Positive lines are transferred to 288J medium to initiate plant regeneration. Following somatic embryo maturation period of 2-4 weeks, well-developed somatic embryos are transferred to 272V medium for germination and then transferred to a lighted culture room. Approximately 7-10 days later, developing plantlets are transferred to 272V medium in tubes for 7-10 days until plantlets are well established. Plants are then transferred to potting soil and grown for 1 week in a growth chamber, and subsequently grown 1-2 weeks in the greenhouse, then grown to maturity.

### Example X. Transformation of Cereal Plants with Expression Vector

A cereal plant, such as corn, wheat, rice, sorghum or barley, can also be transformed with a plasmid vector containing a sequence or polynucleotide of the invention, together with an operably linked constitutive or inducible promoter, to modify a trait or produce ectopic or altered expression. In these cases, a cloning vector, pMEN020 for example, is modified to replace the NptII coding region with the BAR gene of Streptomyces hygroscopicus to confer resistance to phosphinothricin. The KpnI and BgIII sites of the Bar (bialaphos resistance) gene are removed by site-directed mutagenesis with silent codon changes. Preferably, a maize or other plant ubiquitin promoter is inserted in place of the 35S promoter of pMEN020 (see, for example, Christensen et al., Plant Mol. Biol. 12:619-632 (1992); and Christensen, et al., Plant Mol. Biol. 18:675-689 (1992); Christensen et al., Transgenic Res. 5:213-8(1996)). The polypeptide-encoding sequence or cDNA is then inserted downstream of the promoter. Additional expression vector elements can also be inserted, as discussed elsewhere in this document, to optimize expression.

Plasmids according to the present invention may be transformed into corn embryogenic cells derived from immature scutellar tissue by using microprojectile bombardment, with the A188XB73 genotype as the preferred genotype (Fromm et al., Bio/Technology 8: 833-839 (1990); Gordon-Kamm et al., Plant Cell 2: 603-618 (1990)). After microprojectile bombardment the tissues are selected on phosphinothricin to identify the transgenic embryogenic cells (Gordon-Kamm et al., Plant Cell 2: 603-618 (1990)). Transgenic.plants are regenerated by standard corn regeneration techniques (Fromm et al., Bio/Technology 8: 833-839 (1990); Gordon-Kamm et al., Plant Cell 2: 603-618 (1990)).

Various homologs, derivative polypeptides, or polypeptide-encoding polynucleotides can be identified and produced from the information in this document. Any technique available can be used and the examples below are merely exemplary.

To identify exemplary variant or derivative polypeptides, polynucleotides, and homologs of the sequences listed here, many techniques, such as using the BLAST program to screen a public (NCBI for example) or commercial (Incyte for example) sequence databases, screening a cDNA or genomic library by hybridization at low or high stringency, and using PCR techniques using degenerate or non-degenerate primers designed to hybridise against the gene you wish to clone are known in the art. Any GID polynucleotide or cDNA clone can be selected as well as any sequence of the sequence listing. For example, G1073 can be selected. Transgenic plants overexpressing G1073 have the advantageous properties of being large, late flowering, and/or have serrated leaves. The large size and/or late flowering traits would be extremely useful in crops where the vegetative portion of the plant can be commercially harvested (often, vegetative growth stops when plants make the transition to flowering). In this case, it would be advantageous to prevent or delay flowering in order to increase yield or biomass. The plants would also be extremely useful in preparing recombinant therapeutic proteins, such as antibodies or single chain antibodies. Prevention of flowering would also be useful in plants and crops in order to prevent the spread of transgenic pollen and/or to prevent seed set. G1073 can also be used to manipulate leaf shape.

In this example, a homolog of G1073 from Glycine max is identified and a construct expressing this Glycine max cDNA is provided. As noted in the Appendices, the NCBI database is screened using the BLAST algorithm and sequences similar to G1073 are identified, including Glycine max cDNA clones or genomic sequences (BF067277, AW349284 and AI736668).

Using standard techniques, a Glycine max cDNA library is screened using probes derived from the sequence BF067277, AW349284 or AI736668 and a full-length clone is isolated. This full length Glycine max clone can be subcloned into an appropriate expression vector using restriction sites or full-length sequences can be amplified from cDNA or genomic DNA by PCR and subcloned into an appropriate expression vector. Also using standard techniques, a fragment incorporating all or part of the Glycine max sequence, or a fragment of another homolog, is produced with substitution or site-specific mutations. This fragment can be used in PCR amplification to replace all or any of the nucleotides to result in amino acid changes or codon changes. Alternatively, oligos incorporating the substitution change(s) can be used in homologous recombination techniques to replace nucleotides in a sequence. Other available techniques, known in the art, can also be used. Once the sequence differences between any sequence listed or described here to that of a known sequence is displayed, one of skill in the art can use any available method to make one or more substitution changes in the nucleotides or the polypeptides. These changes will preferably result in changes in the amino acid sequence of the encoded polypeptide, creating a derivative or variant polypeptide.

The changes or substitutions can also incorporate preferred codons for a particular species or group of plants. Preferred codons for a number of different plants are known in the art. The changes can also delete or add amino acid residues. One skilled in the art is familiar with a variety of techniques for manipulating a polypeptide-encoding sequence to make one or more changes, substitutions, deletions, or additions, as desired.

As shown here, the sequences listed have homologs in other plant species. Any of the manipulations, procedures for producing transgenic plants, or analysis of the transgenic plants, can be performed using the homolog sequence in place of the specifically listed sequence. Thus, for example, transgenic plants employing the homolog of G1073 from, for example, Lycopersicon esculentum, Medicago truncatula, Oryza sativa, Hordeum vulgare, Glycine max, Lotus japonicus, Solanum tuberosum, Sorghum propinquum, Pinus taeda, Triticum aestivum, Pisum sativu, Antirrhinum majus, Daucus carota, Nicotiana tabacum, Brassica napus, Zea mays, Volvox carteri f. nagariensis, or Chlamydomonas reinhardtii can be used to create plants having ectopic expression or altered expression of the G1073 homolog. Chimeric sequences, employing parts of more than one homolog or parts of a specific sequence, such as G1073, and its homolog(s), can also be created and used. More than one homolog or recombinant polynucleotide can be introduced into a plant to produce a transgenic plant, as known in the art.

All references, publications, patent documents, web pages, links, sequences of Genbank identifiers, sequences of genomic or EST database identifiers, and other documents cited or mentioned herein are hereby incorporated by reference in their entirety for all purposes. Although the invention has been described with reference to specific embodiments and examples, it should be understood that one of ordinary skill can make various modifications without departing from the spirit of the invention. The scope of the invention is not limited to the specific embodiments and examples provided.

The invention is further defined by the following numbered aspects:
1. A transgenic plant comprising a recombinant polynucleotide having a nucleotide sequence selected from the group:
   (a) a nucleotide sequence encoding a polypeptide comprising an amino acid sequence selected from those of SEQ ID NOs.: 2N where N= 1-232, or a complementary nucleotide sequence thereof;
   (b) a nucleotide sequence encoding a polypeptide comprising a conservatively substituted variant of a polypeptide of (a);
   (c) one of SEQ ID NOs.: 2N-1 where N=1-232, or a complementary nucleotide sequence thereof;
   (d) a nucleotide sequence comprising one or more silent substitutions in a nucleotide sequence of (c);
   (e) a nucleotide sequence that hybridizes under stringent conditions to a nucleotide sequence of one or more of: (a), (b), (c), or (d);
   (f) a nucleotide sequence comprising at least 15 consecutive nucleotides outside of a conserved domain of any of (a)-(e);
   (g) a nucleotide sequence comprising a subsequence or fragment of any of (a)-(f), which subsequence or fragment encodes a polypeptide that modifies one or more of a plant's traits;
   (h) a nucleotide sequence having at least 31 % sequence identity to a nucleotide sequence of any of (a)-(g);
   (i) a nucleotide sequence having at least 60% sequence identity to a nucleotide sequence of any of (a)-(g);
   (j) a nucleotide sequence having at least 95% sequence identity to a nucleotide sequence of any of (a)-(g);
   (k) a nucleotide sequence encoding a polypeptide having at least 31% sequence identity outside of a conserved domain of a polypeptide having an amino acid sequence of one of SEQ ID Nos.: 2N where N=1-232;
   (l) a nucleotide sequence encoding a polypeptide having at least 60% sequence identity outside of a conserved domain of a polypeptide having an amino acid sequence of one of SEQ ID Nos.: 2N where N=1-232; or
   (m) a nucleotide sequence encoding a polypeptide having at least 75% sequence identity outside of a conserved domain of a polypeptide having an amino acid sequence of one of SEQ ID Nos.: 2N where N=1-232;
   (n) a nucleotide sequence encoding a polypeptide having at least 95% sequence identity outside of a conserved domain of a polypeptide having an amino acid sequence of one of SEQ ID Nos.: 2N where N=1-232;
   (o) a nucleotide sequence encoding a polypeptide having an amino acid domain with at least 86% sequence identity to a conserved domain of a polypeptide of one of SEQ ID Nos: 2N where N=1-232;
   (p) a nucleotide sequence encoding a polypeptide having an amino acid domain with at least about 90% sequence identity to a conserved domain of a polypeptide of one of SEQ ID Nos: 2N where N=1-232;
   (q) a nucleotide sequence encoding a polypeptide having an amino acid domain with at least about 95% sequence identity to a conserved domain of a polypeptide of one of SEQ ID Nos: 2N where N=1-232;
   (r) a nucleotide sequence encoding a polypeptide having an amino acid domain with at least about 98% sequence identity to a conserved domain of a polypeptide of one of SEQ ID Nos: 2N where N=1-232;
   (s) a nucleotide sequence encoding a polypeptide having at least 31 % sequence identity over the entire length of a polypeptide of one of SEQ ID Nos.: 2N where N=1-232;
   (t) a nucleotide sequence encoding a polypeptide having at least 60% sequence identity over the entire length of a polypeptide of one of SEQ ID Nos.: 2N where N=1-232;
   (u) a nucleotide sequence encoding a polypeptide having at least 75% sequence identity over the entire length of a polypeptide of one of SEQ ID Nos. 2N where N=1-232;
   (v) a nucleotide sequence encoding a polypeptide having at least 95% sequence identity over the entire length of a polypeptide of one of SEQ ID Nos. 2N where N=1-232,
   wherein the plant possesses an altered trait as compared to a wild type or reference plant, or the plant exhibits an altered phenotype as compared to a wild type or reference plant, or the plant exhibits ectopic expression or altered expression of one or more genes associated with a plant trait as compared to a wild type plant.
2. The transgenic plant of aspect 1, further comprising a constitutive, inducible, or tissue-specific promoter operably linked to said recombinant nucleotide.
3. The transgenic plant of aspect 1, wherein the plant is selected from the following group: soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, *Arabidopsis,* spinach, squash, sweet corn, tobacco, tomato, watermelon, mint and other labiates, rosaceous fruits, and vegetable brassicas.
4. An isolated or recombinant polynucleotide having a nucleotide sequence selected from the following group:
   (a) a nucleotide sequence encoding a polypeptide comprising a sequence selected from those of SEQ ID Nos: 2N where N=1-232, or a complementary nucleotide sequence thereof;
   (b) a nucleotide sequence encoding a polypeptide comprising a conservatively substituted variant of a polypeptide of (a);
   (c) one of SEQ ID NOs. 2N-1 where N=1-232, or a complementary nucleotide sequence thereof;
   (d) a nucleotide sequence comprising silent substitutions in a nucleotide sequence of (c);
   (e) a nucleotide sequence that hybridizes under stringent conditions to a nucleotide sequence of one or more of: (a), (b), (c), or (d);
   (f) a nucleotide sequence comprising at least 15 consecutive nucleotides outside of a conserved domain of any of (a)-(e);
   (g) a nucleotide sequence comprising a subsequence or fragment of any of (a)-(f), which subsequence or fragment encodes a polypeptide that modifies one or more of a plant's traits;
   (h) a nucleotide sequence having at least 31 % sequence identity to a nucleotide sequence of any of (a)-(g);
   (i) a nucleotide sequence having at least 60% sequence identity to a nucleotide sequence of any of (a)-(g);
   (j) a nucleotide sequence having at least 95% sequence identity to a nucleotide sequence of any of (a)-(g);
   (k) a nucleotide sequence encoding a polypeptide having at least 31% sequence identity outside of a conserved domain of a polypeptide having an amino acid sequence of one of SEQ ID Nos.: 2N where N=1-232;
   (l) a nucleotide sequence encoding a polypeptide having at least 60% sequence identity outside of a conserved domain of a polypeptide having an amino acid sequence of one of SEQ ID Nos.: 2N where N=1-232; or
   (m) a nucleotide sequence encoding a polypeptide having at least 75% sequence identity outside of a conserved domain of a polypeptide having an amino acid sequence of one of SEQ ID Nos.: 2N where N=1-232;
   (n) a nucleotide sequence encoding a polypeptide having at least 95% sequence identity outside of a conserved domain of a polypeptide having an amino acid sequence of one of SEQ ID Nos.: 2N where N=1-232;
   (o) a nucleotide sequence encoding a polypeptide having an amino acid domain with at least 86% sequence identity to a conserved domain of a polypeptide having an amino acid sequence of one of SEQ ID Nos.: 2N where N=1-232;
   (p) a nucleotide sequence encoding a polypeptide having an amino acid domain with at least about 90% sequence identity to a conserved domain of a polypeptide having an amino acid sequence of one of SEQ ID Nos.: 2N where N=1-232;
   (q) a nucleotide sequence encoding a polypeptide having an amino acid domain with at least about 95% sequence identity to a conserved domain of a polypeptide having an amino acid sequence of one of SEQ ID Nos.: 2N where N=1-232; and
   (r) a nucleotide sequence encoding a polypeptide having an amino acid domain with at least about 98% sequence identity to a conserved domain of a polypeptide having an amino acid sequence of one of SEQ ID Nos.: 2N where N=1-232;
   (s) a nucleotide sequence encoding a polypeptide having at least 31 % sequence identity over the entire length of a polypeptide having an amino acid sequence of one of SEQ ID Nos.: 2N where N=1-232;
   (t) a nucleotide sequence encoding a polypeptide having at least 60% sequence identity over the entire length of a polypeptide having an amino acid sequence of one of SEQ ID Nos.: 2N where N=1-232;
   (u) a nucleotide sequence encoding a polypeptide having at least 75% sequence identity over the entire length of a polypeptide having an amino acid sequence of one of SEQ ID Nos.: 2N where N=1-232;
   (v) a nucleotide sequence encoding a polypeptide having at least 95% sequence identity over the entire length of a polypeptide having an amino acid sequence of one of SEQ ID Nos.: 2N where N=1-232.
5. The isolated or recombinant polynucleotide of aspect 4, further comprising a constitutive, inducible, or tissue-specific promoter operably linked to the polynucleotide nucleotide.
6. An isolated or recombinant polypeptide comprising a subsequence of at least about 15 contiguous amino acids encoded by the recombinant or isolated polynucleotide of aspect 4.
7. A method of using the isolated or recombinant polynucleotide of aspect 4 for producing a plant having a modified trait, the method comprising selecting a polynucleotide that encodes a polypeptide, inserting the polynucleotide into an expression vector, introducing the vector into a plant or a cell of a plant to overexpress the polypeptide, thereby producing a modified plant, and selecting for a modified trait.
8. The transgenic plant of aspect 1, wherein the trait is selected from the group: enhanced tolerance to freezing; enhanced tolerance to chilling; enhanced tolerance to heat; enhanced tolerance to drought; enhanced tolerance to water saturation; enhanced tolerance to radiation; enhanced tolerance to ozone; enhanced tolerance to microbial disease; enhanced tolerance to fungal disease; enhanced tolerance to viral disease; enhanced tolerance to pest infestation; decreased herbicide sensitivity; enhanced tolerance to heavy metals; enhanced ability to take up heavy metals; and enhanced growth under poor photoconditions.
9. The transgenic plant of aspect 1, wherein the trait is an alteration in the level of one or more of the compounds selected from the group: taxol, tocopherol, tocotrienol, sterols, phytosterols, vitamins, wax monomers, anti-oxidants, amino acids, lignins, cellulose, tannins, prenyllipids, glucosinolates, and terpenoids.
10. The transgenic plant of aspect 1, wherein the trait is an alteration in one or more physical characteristics selected from the group: number oftrichomes; fruit and seed size and number; yield of stems; yield of leaves; yield of roots; stability of seeds during storage; susceptibility of the seed to shattering; root hair length; root hair quantity; internode distances; and the quality of seed coat.
11. The transgenic plant of aspect 1, wherein the trait is an alteration in a plant growth characteristic selected from the group: growth rate; germination rate of seeds; vigor of plants; vigor of seedlings; leaf senescence; flower senescence; male sterility; apomixis; flowering time; flower abscission; rate of nitrogen uptake; biomass; transpiration characteristics; apical dominance; branching pattern; number of organs; organ identity; organ shape; and organ size.
12. The transgenic plant of aspect 1, wherein the trait is an alteration in one or more characteristics selected from the group: protein production; oil production; seed protein production; seed oil production; insoluble sugar level; soluble sugar level; and starch composition.
13. The method of aspect 7, wherein the trait is selected from the group: enhanced tolerance to freezing; enhanced tolerance to chilling; enhanced tolerance to heat; enhanced tolerance to drought; enhanced tolerance to water saturation; enhanced tolerance to radiation; enhanced tolerance to ozone; enhanced tolerance to microbial disease; enhanced tolerance to fungal disease; enhanced tolerance to viral disease; enhanced tolerance to pest infestation; decreased herbicide sensitivity; enhanced tolerance to heavy metals; enhanced ability to take up heavy metals; and enhanced growth under poor photoconditions.
14. The method of aspect 7, wherein the trait is an alteration in the level of one or more of the compounds selected from the group: taxol, tocopherol, tocotrienol, sterols, phytosterols, vitamins, wax monomers, anti-oxidants, amino acids, lignins, cellulose, tannins, prenyllipids, glucosinolates, and terpenoids.
15. The method of aspect 7, wherein the trait is an alteration in one or more physical characteristics selected from the group: number oftrichomes; fruit and seed size and number; yield of stems; yield of leaves; yield of roots; stability of seeds during storage; susceptibility of the seed to shattering; root hair length; root hair quantity; internode distances; and the quality of seed coat.
16. The method of aspect 7, wherein the trait is an alteration in a plant growth characteristic selected from the group: growth rate; germination rate of seeds; vigor of plants; vigor of seedlings; leaf senescence; flower senescence; male sterility; apomixis; flowering time; flower abscission; rate of nitrogen uptake; biomass; transpiration characteristics; apical dominance; branching pattern; number of organs; organ identity; organ shape; and organ size.
17. The method of aspect 7, wherein the trait is an alteration in one or more characteristics selected from the group: protein production; oil production; seed protein production; seed oil production; insoluble sugar level; soluble sugar level; and starch composition.
18. A plant produced by the method of aspect 13.
19. A plant produced by the method of aspect 14.
20. A plant produced by the method of aspect 15.
21. A plant produced by the method of aspect 16.
22. A plant produced by the method of aspect 17.
23. A method of using the isolated or recombinant polynucleotide of aspect 4 for producing a plant having a modified trait, the method comprising selecting a polynucleotide that when expressed produces an antisense nucleic acid, inserting the polynucleotide into an expression vector, introducing the vector into a plant or a cell of a plant to express the antisense nucleic acid, thereby producing a modified plant, and selecting for a modified trait.
24. The method of aspect 23, wherein the trait is selected from the group: enhanced tolerance to freezing; enhanced tolerance to chilling; enhanced tolerance to heat; enhanced tolerance to drought; enhanced tolerance to water saturation; enhanced tolerance to radiation; enhanced tolerance to ozone; enhanced tolerance to microbial disease; enhanced tolerance to fungal disease; enhanced tolerance to viral disease; enhanced tolerance to pest infestation; decreased herbicide sensitivity; enhanced tolerance to heavy metals; enhanced ability to take up heavy metals; and enhanced growth under poor photoconditions.
25. The method of aspect 23, wherein the trait is an alteration in the level of one or more of the compounds selected from the group: taxol, tocopherol, tocotrienol, sterols, phytosterols, vitamins, wax monomers, anti-oxidants, amino acids, lignins, cellulose, tannins, prenyllipids, glucosinolates, and terpenoids.
26. The method of aspect 23, wherein the trait is an alteration in one or more physical characteristics selected from the group consisting of: the number oftrichomes, fruit and seed size and number, yield of stems, leaves, or roots, stability of seeds during storage, susceptibility of the seed to shattering, root hair length and quantity, internode distances, or the quality of seed coat.
27. The method of aspect 23, wherein the trait is an alteration in a plant growth characteristic selected from the group: growth rate; germination rate of seeds; vigor of plants; vigor of seedlings; leaf senescence; flower senescence; male sterility; apomixis; flowering time; flower abscission; rate of nitrogen uptake; biomass; transpiration characteristics; apical dominance; branching pattern; number of organs; organ identity; organ shape; and organ size.
28. The method of aspect 23, wherein the trait is an alteration in one or more characteristics selected from the group: protein production; oil production; seed protein production; seed oil production; insoluble sugar level; soluble sugar level; and starch composition.
29. A plant produced by the method of aspect 24.
30. A plant produced by the method of aspect 25.
31. A plant produced by the method of aspect 26.
32. A plant produced by the method of aspect 27.
33. A plant produced by the method of aspect 28.
34. An isolated or recombinant polypeptide comprising a subsequence of at least about 10 0 contiguous amino acids encoded by the recombinant or isolated polynucleotide of aspect 4, wherein the contiguous amino acids are outside of a conserved domain.
35. An isolated or recombinant polypeptide comprising a subsequence of at least about 20 contiguous amino acids encoded by the recombinant or isolated polynucleotide of aspect 4, wherein the contiguous amino acids are outside of a conserved domain.
36. An isolated or recombinant polypeptide comprising a subsequence of at least about 30 contiguous amino acids encoded by the recombinant or isolated polynucleotide of aspect 4, wherein the contiguous amino acids are outside of a conserved domain.
37. An isolated or recombinant polypeptide comprising a subsequence of at least about 10 contiguous amino acids encoded by the recombinant or isolated polynucleotide of aspect 4, wherein the contiguous amino acids are within a conserved domain.
38. An isolated or recombinant polypeptide comprising a subsequence of at least about 20 contiguous amino acids encoded by the recombinant or isolated polynucleotide of aspect 4, wherein the contiguous amino acids are within a conserved domain.
39. An isolated or recombinant polypeptide comprising a subsequence of at least about 30 contiguous amino acids encoded by the recombinant or isolated polynucleotide of aspect 4, wherein the contiguous amino acids are within a conserved domain.
40. An isolated or recombinant polypeptide having at least 31 % sequence identity over the entire length of a polypeptide having an amino acid sequence of one of SEQ ID Nos.: 2N where N=1-232, or the length of the polypeptide itself.
41. An isolated or recombinant polypeptide having at least 60% sequence identity over the entire length of a polypeptide having an amino acid sequence of one of SEQ ID Nos.: 2N where N=1-232, or the length of the polypeptide itself.
42. An isolated or recombinant polypeptide having at least 75% sequence identity over the entire length of a polypeptide having an amino acid sequence of one of SEQ ID Nos.: 2N where N=1-232, or the length of the polypeptide itself.
43. An isolated or recombinant polypeptide having at least 95% sequence identity over the entire length of a polypeptide having an amino acid sequence of one of SEQ ID Nos.: 2N where N=1-232, or the length of the polypeptide itself.
44. An isolated or recombinant polynucleotide having the sequence one of SEQ ID NOs.: 2N-1 I where N=1-232, or a complementary nucleotide sequence thereof..
45. The polynucleotide of aspect 44, which has the sequence of one of SEQ ID Nos.: 1, 3, 5, 7, 9, 11, 13, 15, 17, 19, 21, 23, 25, 27, 29, 31, 33, 35, or 37.
46. The polynucleotide of aspect 44, which has the sequence of one of SEQ ID Nos.: 53, 79, 81, 107, 125, 153, 167, 203, 223, 289, 285, or 287.
47. The polynucleotide of aspect 44, which has the sequence of one of SEQ ID Nos.: 313, 345, 365, 395, 417, 425, 435, 455, 457, 459, 461, or 463.
48. A computer readable medium having stored sequence information comprising the polynucleotide sequence of aspect 44.
49. The computer readable medium of aspect 48, having stored sequence information comprising the sequence of one of SEQ ID Nos.: 1-37.
50. The computer readable medium of aspect 48, having stored sequence information comprising the sequence of one of SEQ ID Nos.: 53, 54, 79, 80, 81, 82, 107, 108, 125, 126, 153, 154, 167, 168, 203, 204, 223, 224, 289, 290, 285, 286, 287, or 288.
51. The computer readable medium of aspect 48, having stored sequence information comprising the polynucleotide sequence of one of SEQ ID Nos.: 313, 314, 345, 346, 365, 366, 395, 396, 461, 462, 417, 418, 425, 426, 435, 436, 455, 456, 457, 458, 459, 460, 461, 462, 463, or 464.
52. A method of identifying a homolog sequence from a database comprising a plurality of known plant sequences, the method comprising: inputting sequence information selected from one or more of SEQ ID Nos. 1-464; and querying the database to identify a homolog sequence.
53. The method of aspect 52, wherein the database being queried comprises a database of known genomic, cDNA, EST, or protein sequences.
54. The method of aspect 52, wherein inputting sequence information comprises copying the sequence information from a CD.
55. The method of aspect 52, wherein the sequence data comprises one of SEQ ID Nos.: 1-37.
56. The method of aspect 52, wherein the sequence data comprises one of SEQ ID Nos.: 53, 54, 79, 80, 81, 82, 107, 108, 125, 126, 153, 154, 167, 168, 203, 204, 223, 224, 289, 290, 285, 286, 287, or 288.
57. The method of aspect 52, wherein the sequence data comprises of SEQ ID Nos.: 313, 314, 345, 346, 365, 366, 395, 396, 461, 462, 417, 418, 425, 426, 435, 436, 455, 456, 457, 458, 459, 460, 461, 462, 463, or 464.
58. The method of aspect 52, wherein the sequence data comprises a 20 nucleotide region or 6 amino acid region of one of SEQ ID Nos.: 1-37.
59. The method of aspect 52, wherein the sequence data comprises a 20 nucleotide region or 6 amino acids region one of SEQ ID Nos.: 53, 54, 79, 80, 81, 82, 107, 108, 125, 126, 153, 154, 167, 168, 203, 204, 223, 224, 289, 290, 285, 286, 287, or 288.
60. The method of aspect 52, wherein the sequence data comprises a 20 nucleotide region or 6 amino acid region of one of SEQ ID Nos.: 313, 314, 345, 346, 365, 366, 395, 396, 461, 462, 417, 418, 425, 426, 435, 436, 455, 456, 457, 458, 459, 460, 461, 462, 463, or 464.
61. A homolog identified by the method of aspect 52.
62. The homolog of aspect 61, identified by the method of aspect 53.
63. The homolog of aspect 61, identified by the method of aspect 54.
64. The homolog of aspect 61, identified by the method of aspect 55.
65. The homolog of aspect 61, identified by the method of aspect 55.
66. The homolog of aspect 61, identified by the method of aspect 56.
67. The homolog of aspect 61, identified by the method of aspect 57.
68. The homolog of aspect 61, identified by the method of aspect 5 8.
69. The homolog of aspect 61, identified by the method of aspect 59.
70. The homolog of aspect 61, identified by the method of aspect 60.

## Claims

1. A transgenic plant, or progeny thereof, wherein said plant comprises an expression vector or cassette for expression of the polypeptide of SEQ ID NO:126 or a homologue thereof having at least 50% amino acid identity to SEQ ID NO:126, wherein expression of said polypeptide provides the trait of late flowering in said plant.

2. The transgenic plant of claim 1, wherein said late flowering provides a plant that has increased yield or biomass.

3. The transgenic plant of claim 1 or 2 wherein said polypeptide has at least 75% amino acid identity to SEQ ID NO:126.

4. The transgenic plant of claim 1 or 2 wherein said polypeptide has at least 90% amino acid identity to SEQ ID NO:126.

5. The transgenic plant of any one of claims 1-4 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

6. A seed of the transgenic plant of any of claims 1-5 that contains an expression vector or cassette encoding said nucleotide sequence encodes the transcription factor polypeptide of SEQ ID NO:126 or a homologue thereof having at least 50% amino acid identity to SEQ ID NO:126 wherein expression of said polypeptide provides the trait of late flowering in said plant.

7. A plant part, plant tissue or plant cells obtained from the transgenic plant of any of claims 1 to 5, said plant part or material comprising the expression vector or cassette as defined in claim 1, 2 or 3.

8. A method for producing transgenic plant having the trait of late flowering, said method comprising:
producing a transgenic plant having an expression vector or cassette comprising a nucleotide sequence that encodes the transcription factor polypeptide of SEQ ID NO:126 or a homologue thereof having at least 50% amino acid identity to SEQ ID NO:126 wherein overexpression of said polypeptide provides the trait of increased seed protein; and
selecting a plant having said trait of late flowering in said plant caused by expression of said polypeptide.

9. The method of claim 8, wherein said late flowering provides a plant that has increased yield or biomass.

10. The method of claim 8 or 9 wherein said nucleotide sequence that encodes a polypeptide is operably linked to a constitutive, inducible or tissue-active promoter.

11. The method of any one of claims 8 to 10, wherein said polypeptide has at least 75% amino acid identity to SEQ ID NO:126.

12. The method of any one of claims 8 to 10, wherein said polypeptide has at least 90% amino acid identity to SEQ ID NO:126.

13. The method of any one of claims 7 to 10 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

14. A method of preparing a therapeutic protein such as an antibody or single chain antibody, said method comprising production of said protein in a plant with delayed flowering.

15. The method of claim 14 wherein said plant is the transgenic plant of any one of claims 1-5.
